# EUROPEAN PATENT SPECIFICATION

(11) **EP 2 383 252 B1**
(45) Date of publication and mention of the grant of the patent: **25.06.2014**
(21) Application number: 09833432.9
(22) Date of filing: 15.12.2009
(51) Int. Cl.: C07C 67/307, C07C 69/63, C07D 207/16, C07C 67/31

(54) **METHOD FOR MANUFACTURING HYDROXYL GROUP SUBSTITUTION PRODUCT**
VERFAHREN ZUR HERSTELLUNG EINES HYDROXYLGRUPPENSUBSTITUTIONSPRODUKTS
PROCÉDÉ DE FABRICATION D'UN PRODUIT SUBSTITUÉ PAR UN GROUPE HYDROXYLE

(30) Priority: 17.12.2008 JP 2008321502; 30.09.2009 JP 2009225952
(43) Date of publication of application: 02.11.2011
(73) Proprietor: Central Glass Company, Limited, Yamaguchi 755-0001 (JP)
(72) Inventor: ISHII, Akihiro, Saitama 350-1151 (JP); YASUMOTO, Manabu, Saitama 350-1151 (JP); UEDA, Koji, Saitama 350-1151 (JP)
(74) Representative: Manitz, Finsterwald & Partner GbR
(86) International application number: PCT/JP2009/070904
(87) International publication number: WO 2010/071129

(56) References cited:
- WO-A1-99/55666
- WO-A1-2006/098444
- WO-A1-2008/001718
- WO-A1-2008/090755
- DATABASE REAXYS [Online] Elsevier Properties SA; 2012, XP002675996, Database accession no. 1975449 & Hedayatullah, Mir; Guy, Alain; Denivelle, Leon: "Synthese de Fluorosulfates D'Aryle Encombres" In: "Phosphorus and Sulfur and the Related Elements", 1980 vol. 8, page 125-126,

## Description

The present invention relates to a method for manufacturing a hydroxyl group substitution product, which is important as intermediates for pharmaceutical and agricultural chemicals.

### Background Art

Hydroxyl group substitution products are important as intermediates for pharmaceutical and agricultural chemicals. There have been disclosed, as conventional manufacturing techniques relevant to the present invention, a Mitsunobu reaction process that uses diethyl azodicarboxylate (DEAD) and triphenyl phosphine (Patent Document 1) and a process that forms a mesyloxy group as a leaving group and then reacts the mesyloxy group with a nucleophile (Non-Patent Document 1).

The present applicant has disclosed a process for dehydroxyfluorination of an alcohol by the combined use of an organic base and sulfuryl fluoride (SO₂F₂) (Patent Document 2).

### Prior Art Documents

### Patent Documents

Patent Document 1: Published Japanese Translation of PCT Application No. 2007-537300
Patent Document 2: Japanese Laid-Open Patent Publication No. 2006-290870

### Non-Patent Documents

Non-Patent Document 1: Bioorganic & Medicinal Chemistry (U.S.), 2008, Vol.16, P.578-585

### Disclosure of the Invention

It is an object of the present invention to provide an industrial manufacturing method of a hydroxyl group substitution product. In order to achieve the object of the present invention, it is necessary to solve the following problems in the prior art techniques.

The process of Patent Document 1 needs to use diethyl azodicarboxylate and triphenyl phosphine, which are relatively expensive for large-scale applications. Further, the process of Patent Document 1 stoichiometrically generates a by-product difficult to separate from the target compound and thus requires complicated purification operation such as column chromatography.

The process of Non-Patent Document 1 proceeds in two process steps by way of a reactive intermediate and presents problems such as process complication and increase in waste associated with such process complication.

The process of Patent Document 2 generates a fluorosulfuric acid ester and a fluorine anion (F⁻) in the reaction system. As the fluorosulfuric acid ester undergoes substitution reaction with the fluorine anion very rapidly, it has been totally unknown whether the target hydroxyl group substitution product of the present invention can be obtained selectively in preference to the fluorination product even when the reaction process is conducted in the presence of any nucleophile (X⁻) other than the fluorine anion as in the present invention (see Scheme 1).

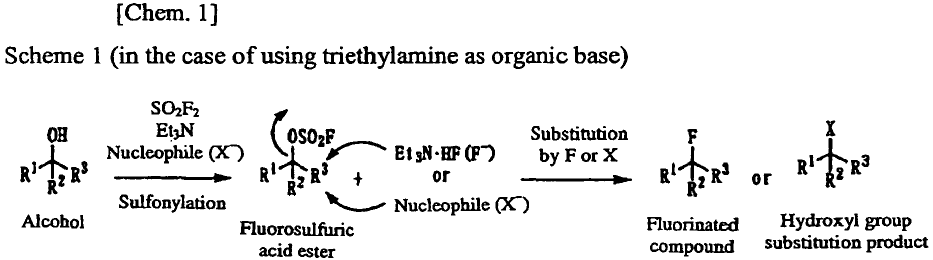

As mentioned above, there has been a strong demand for an industrial manufacturing method that uses a relatively cheap reagent suitable for large-scale applications and can be accomplished in a simple process with easy purification operation and less waste generation.

The present inventors have made extensive researches in view of the above problems and, as a result, have found that it is possible to manufacture a hydroxyl group substitution product by reaction of an alcohol with sulfuryl fluoride in the presence of a specific nucleophile and an organic base. As the raw substrate, preferred are optically active alcohols, more preferably an optically active α-hydroxyester and an optically active 4-hydroxyproline, as the resulting optically active hydroxyl group substitution products (notably, optically active α-hydroxyl group substitution ester and optically active 4-hydroxyl group substitution proline) are very important as intermediates for pharmaceutical and agricultural chemicals.

The manufacturing conditions of the present invention are similar to the dehydroxyfluorination reaction conditions of Patent Document 2. The present inventors have however found that, when the reaction is conducted in the presence of the specific nucleophile other than fluorine anion, it is possible that the hydroxyl group substitution product derived from the nucleophile can be obtained selectively in preference to fluorinated compounds. In the present invention, the nucleophile is a monovalent anion represented by X⁻. As a species X that constitutes the nucleophile X⁻, there can be used a halogen atom such as chlorine, bromine or iodine, an azide group, a nitrooxy group, a cyano group, a thiocyanate group, a formyloxy group, an alkylcarbonyloxy group, a substituted alkylcarbonyloxy group, an arylcarbonyloxy group and a substituted arylcarbonyloxy group. Among others, a halogen atom such as chlorine, bromine or iodine, an azide group, a formyloxy group, an alkylcarbonyloxy group, a substituted alkylcarbonyloxy group, an arylcarbonyloxy group and a substituted arylcarbonyloxy group are preferred. Particularly preferred are a halogen atom such as chlorine, bromine or iodine and an azide group. The desired reaction proceeds very favorably though the use of such a nucleophile.

In this way, the present inventors have found the very useful techniques for manufacturing of the hydroxyl group substitution product. The present invention is based on these findings.

Namely, the present invention provides an industrial method for manufacturing a hydroxyl group substitution product as set forth below in Inventive Aspects 1 to 4.

### [Inventive Aspect 1]

A method for manufacturing a hydroxyl group substitution product of the general formula [2], comprising: reacting an alcohol of the general formula [1] with sulfuryl fluoride (SO₂F₂) in the presence of an organic base and a nucleophile (X⁻), 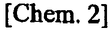 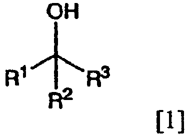 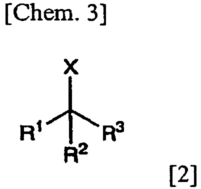 where R¹, R² and R³ each independently represent a hydrogen atom, an alkyl group, a substituted alkyl group, an alkenyl group, a substituted alkenyl group, an alkynyl group, a substituted alkynyl group, an aromatic ring group, a substituted aromatic ring group, a formyl group, an alkylcarbonyl group, a substituted alkylcarbonyl group, an arylcarbonyl group, a substituted arylcarbonyl group, an alkoxycarbonyl group, a substituted alkoxycarbonyl group, aminocarbonyl group, an alkylaminocarbonyl group, a substituted alkylaminocarbonyl group, an arylaminocarbonyl group, a substituted arylaminocarbonyl group or a cyano group; when two of R¹, R² and R³ are any substituent groups other than hydrogen atom, formyl group, aminocarbonyl group and cyano group, these two substituent groups may form a ring structure by a covalent bond between carbon atoms thereof through or without a heteroatom; and X represents a halogen atom selected from the group consisting of chlorine, bromine and iodine, an azide group, a nitrooxy group, a cyano group, a thiocyanate group, a formyloxy group, an alkylcarbonyloxy group, a substituted alkylcarbonyloxy group, an arylcarbonyloxy group or a substituted arylcarbonyloxy group, wherein, if any of R¹, R² and R³ is a substituted alkyl group, a substituted alkenyl group, a substituted alkynyl group, a substituted aromatic ring group, a a substituted alkylcarbonyl group, a substituted arylcarbonyl group, a substituted alkoxycarbonyl group, a substituted alkylaminocarbonyl group or a substituted arylaminocarbonyl group or if X is a substituted alkylcarbonyloxy group any of the carbon atoms may be substituted, and the substituent is selected from the group consisting of halogen atoms; an azide group; a nitro group; lower alkyl groups; lower haloalkyl groups; lower alkoxy groups; lower haloalkoxy groups; lower alkylamino groups; lower alkylthio groups; a cyano group; lower alkoxycarbonyl groups; an aminocarbonyl group; lower alkylaminocarbonyl groups; lower alkenyl groups; lower alkynyl groups; aromatic ring groups; aromatic ring oxy groups; aliphatic heterocyclic groups; protected hydroxyl groups; protected amino groups; protected thiol groups; an aldehyde group; protected aldehyde groups; protected carboxyl groups and combinations thereof, wherein the term "lower" means that the group to which the term is attached has 1 to 6 carbon atoms in the form of a linear structure, a branched structure or a cyclic structure in the case of 3 or more carbon atoms.

### [Inventive Aspect 2]

A method for manufacturing an optically active hydroxyl group substitution product of the general formula [4], comprising: reacting an optically active alcohol of the general formula [3] with sulfuryl fluoride (SO₂F₂) in the presence of an organic base and a nucleophile (Y⁻), 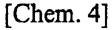 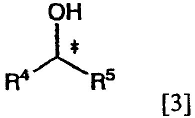 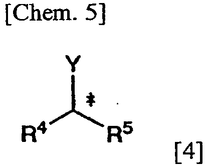 where R⁴ and R⁵ each independently represent an alkyl group, a substituted alkyl group, a formyl group, an alkylcarbonyl group, a substituted alkylcarbonyl group, an arylcarbonyl group, a substituted arylcarbonyl group, an alkoxycarbonyl group, a substituted alkoxycarbonyl group, an aminocarbonyl group, an alkylaminocarbonyl group, a substituted alkylaminocarbonyl group, an arylaminocarbonyl group, a substituted arylaminocarbonyl group or a cyano group; R⁴ and R⁵ are in kind different from each other; when R⁴ and R⁵ represent any two substituent groups other than formyl group, aminocarbonyl group and cyano group, these two substituent groups may form a ring structure by a covalent bond between carbon atoms thereof through or without a heteroatom; Y represents a halogen atom selected from the group consisting of chlorine, bromine and iodine, an azide group, a formyloxy group, an alkylcarbonyloxy group, a substituted alkylcarbonyloxy group, an arylcarbonyloxy group or a substituted arylcarbonyloxy group; * represents an asymmetric carbon atom; and the configuration of the asymmetric carbon atom is inverted in the reaction, wherein, if any of R⁴ and R⁵ is a substituted alkyl group, a substituted alkylcarbonyl group, a substituted arylcarbonyl group, a substituted alkoxycarbonyl group, a substituted alkylaminocarbonyl group or a substituted arylaminocarbonyl group or if Y is a substituted alkylcarbonyloxy group or a substituted arylcarbonyloxy group any of the carbon atoms may be substituted, and the substituent is selected from the group consisting of halogen atoms; an azide group; a nitro group; lower alkyl groups; lower haloalkyl groups; lower alkoxy groups; lower haloalkoxy groups; lower alkylamino groups; lower alkylthio groups; a cyano group; lower alkoxycarbonyl groups; an aminocarbonyl group; lower alkylaminocarbonyl groups; lower alkenyl groups; lower alkynyl groups; aromatic ring groups; aromatic ring oxy groups; aliphatic heterocyclic groups; protected hydroxyl groups; protected amino groups; protected thiol groups; an aldehyde group; protected aldehyde groups; protected carboxyl groups and combinations thereof, wherein the term "lower" means that the group to which the term is attached has 1 to 6 carbon atoms in the form of a linear structure, a branched structure or a cyclic structure in the case of 3 or more carbon atoms.

### [Inventive Aspect 3]

A method for manufacturing an optically active α-hydroxyl group substitution ester of the general formula [6], comprising: reacting an optically active α-hydroxyester of the general formula [5] with sulfuryl fluoride (SO₂F₂) in the presence of an organic base and a nucleophile (Z⁻), 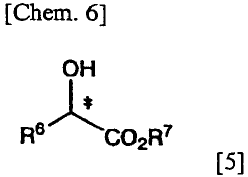 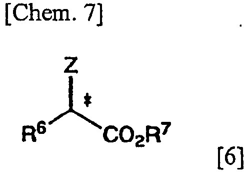 where R⁶ and R⁷ each independently represent an alkyl group or a substituted alkyl group and may form a ring structure by a covalent bond between carbon atoms thereof through or without a heteroatom; Z represents a halogen atom selected from the group consisting of chlorine, bromine and iodine or an azide group; * represents an asymmetric carbon atom; and the configuration of the asymmetric carbon atom is inverted in the reaction, wherein, if any of R⁶ and R⁷ is a substituted alkyl group, the substituent is selected from the group consisting of halogen atoms; an azide group; a nitro group; lower alkyl groups; lower haloalkyl groups; lower alkoxy groups; lower haloalkoxy groups; lower alkylamino groups; lower alkylthio groups; a cyano group; lower alkoxycarbonyl groups; an aminocarbonyl group; lower alkylaminocarbonyl groups; lower alkenyl groups; lower alkynyl groups; aromatic ring groups; aromatic ring oxy groups; aliphatic heterocyclic groups; protected hydroxyl groups; protected amino groups; protected thiol groups; an aldehyde group; protected aldehyde groups; protected carboxyl groups and combinations thereof, wherein the term "lower" means that the group to which the term is attached has 1 to 6 carbon atoms in the form of a linear structure, a branched structure or a cyclic structure in the case of 3 or more carbon atoms .

### [Inventive Aspect 4]

A method for manufacturing an optically active 4-hydroxyl group substitution proline of the general formula [8], comprising: reacting an optically active 4-hydroxyproline of the general formula [7] with sulfuryl fluoride (SO₂F₂) in the presence of an organic base and a nucleophile (Z⁻), 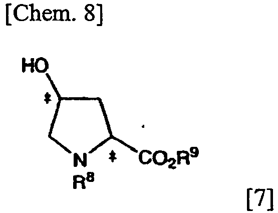 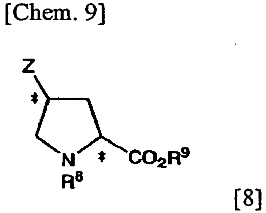 where R⁸ represents a secondary amino protecting group; R⁹ represents a carboxyl protecting group; Z represents a halogen atom selected from the group consisting of chlorine, bromine and iodine or an azide group; and * each represent an asymmetric carbon atom; the configuration of the asymmetric carbon atom at 2-position is maintained throughout the reaction; and the configuration of the asymmetric carbon atom at 4-position is inverted in the reaction.

### Detailed Description

The advantages of the present invention over the prior art techniques will be explained below.

In the present invention, sulfuryl fluoride is used as a reagent. This reagent is widely adapted as a fumigant and is available at low cost for large-scale applications. Further, it is possible in the present invention to achieve a simple reaction process with easy purification operation and less waste generation as the reaction proceeds in a single process step and generates no by-product difficult to separate from the target compound. It is also possible that, by the use of the alcohol substrate of high optical purity, the hydroxyl group substitution product of high optical purity can be obtained upon inversion of the configuration of the asymmetric carbon atom as the configuration of the asymmetric carbon atom is highly inverted in the reaction.

As mentioned above, the manufacturing method of the present invention solves all of the prior art problems and can be applied for industrial uses.

The manufacturing method of the hydroxyl group substitution product according to the present invention will be described in detail below.

In the present invention, a hydroxyl group substitution product of the general formula [2] is manufactured by reaction of an alcohol of the general formula [1] with sulfuryl fluoride in the presence of an organic base and a nucleophile.

In the alcohol of the general formula [1], R¹, R² and R³ each independently represents a hydrogen atom, an alkyl group, a substituted alkyl group, an alkenyl group, a substituted alkenyl group, an alkynyl group, a substituted alkynyl group, an aromatic ring group, a substituted aromatic ring group, a formyl group, an alkylcarbonyl group, a substituted alkylcarbonyl group, an arylcarbonyl group, a substituted arylcarbonyl group, an alkoxycarbonyl group, a substituted alkoxycarbonyl group, an aminocarbonyl group, an alkylaminocarbonyl group, a substituted alkylaminocarbonyl group, an arylaminocarbonyl group, a substituted arylaminocarbonyl group or a cyano group. The alcohol is preferably an optically active alcohol in which one of the three substituent groups is an hydrogen atom and two of the three substituent groups are different in kind from each other and are each independently an alkyl group, a substituted alkyl group, a formyl group, an alkylcarbonyl group, a substituted alkylcarbonyl group, an arylcarbonyl group, a substituted arylcarbonyl group, an alkoxycarbonyl group, a substituted alkoxycarbonyl group, an aminocarbonyl group, an alkylaminocarbonyl group, a substituted alkylaminocarbonyl group, an arylaminocarbonyl group, a substituted arylaminocarbonyl group or a cyano group. Particularly preferred are: an optically active α-hydroxyester in which one of the three substituent groups is a hydrogen atom, another one of the three substituent groups is an alkoxycarbonyl group or a substituted alkoxycarbonyl group and the other one of the three substituent group is an alkyl group or a substituted alkyl group; and an optically active 4-hydroxyproline in which a secondary amino group and an carboxyl group are protected by protecting groups, respectively.

The alkyl group can have 1 to 18 carbon atoms and can be in the form of a linear or branched structure, or a cyclic structure (in the case of 3 or more carbon atoms). The alkenyl group refers to a group in which any number of single bonds between any two adjacent carbon atoms of the above alkyl group has been replaced with a double bond. In the alkenyl group, the double bond can be in an E-configuration, a Z-configuration or a mixture thereof (the alkenyl carbon (SP² carbon) may not be linked directly to the carbon to which the hydroxyl group is bonded). The alkynyl group refers to a group in which any number of single bonds between any two adjacent carbon atoms of the above alkyl group has been replaced with a triple bond (the alkynyl carbon (SP carbon) may not be linked directly to the carbon to which the hydroxyl group is bonded). The aromatic ring group can have 1 to 18 carbon atoms and can be in the form of an aromatic hydrocarbon group, such as phenyl, naphthyl or anthryl, or an aromatic heterocyclic group containing a heteroatom e.g. nitrogen, oxygen or sulfur, such as pyrrolyl, furyl, thienyl, indolyl, benzofuryl or benzothienyl. The formyl group refers to a group represented by -COH. The alkyl moiety (R) of the alkylcarbonyl group (-COR) has the same definition as that of the above alkyl group. The aryl moiety (Ar) of the arylcarbonyl group (-COAr) has the same definition as that of the above aromatic ring group. The alkyl moiety (R) of the alkoxycarbonyl group (-CO₂R) has the same definition as that the above alkyl group. The aminocarbonyl group refers to a group represented by -CONH₂. The alkyl moiety (R) of the alkylaminocarbonyl group (-CONHR or -CONR₂) has the same definition as that of the above alkyl group. The aryl moiety (Ar) of the arylaminocarbonyl group (-CONHAr or -CONAr₂) has the same definition as that of the above aromatic ring group.

Any of the carbon atoms of the alkyl group, the alkenyl group, the alkynyl group, the aromatic ring group, the alkylcarbonyl group, the arylcarbonyl group, the alkoxycarbonyl group, the alkylaminocarbonyl group and the arylaminocarbonyl group may be substituted with any number of and any combination of substituents (which correspond to the substituted alkyl group, the substituted alkenyl group, the substituted alkynyl group, the substituted aromatic ring group, the substituted alkylcarbonyl group, the substituted arylcarbonyl group, the substituted alkoxycarbonyl group, the substituted alkylaminocarbonyl group and the substituted arylaminocarbonyl group, respectively). Examples of such substituents are: halogen atoms such as fluorine, chlorine, bromine and iodine; azide group; nitro group; lower alkyl groups such as methyl, ethyl and propyl; lower haloalkyl groups such as fluoromethyl, chloromethyl and bromomethyl; lower alkoxy groups such as methoxy, ethoxy and propoxy; lower haloalkoxy groups such as fluoromethoxy, chloromethoxy and bromomethoxy; lower alkylamino groups such as dimethylamino, diethylamino and dipropylamino; lower alkylthio groups such as methylthio, ethylthio and propylthio; cyano group; lower alkoxycarbonyl groups such as methoxycarbonyl, ethoxycarbonyl and propoxycarbonyl; aminocarbonyl group; lower alkylaminocarbonyl groups such as dimethylaminocarbonyl, diethylaminocarbonyl and dipropylaminocarbonyl; unsaturated groups such as lower alkenyl groups and lower alkynyl groups; aromatic ring groups such as phenyl, naphthyl, pyrrolyl, furyl and thienyl; aromatic ring oxy groups such as phenoxy, naphthoxy, pyrrolyloxy, furyloxy and thienyloxy; aliphatic heterocyclic groups such as piperidyl, piperidino and morpholinyl; hydroxyl group; protected hydroxyl groups; amino group; protected amino groups (including amino acids and peptide residues); thiol group; protected thiol groups; aldehyde group; protected aldehyde groups; carboxyl group; and protected carboxyl groups.

The following terms are herein defined by the following meanings in the present specification. The term "lower" means that the group to which the term is attached has 1 to 6 carbon atoms in the form of a linear structure, a branched structure or a cyclic structure (in the case of 3 or more carbon atoms). It means that, when the "unsaturated group" is a double bond (alkenyl group), the double bond can be in an E-configuration, a Z-configuration or a mixture thereof. The "protected hydroxyl, amino, thiol, aldehyde and carboxyl groups" may refer to those having protecting groups as described in "Protective Groups in Organic Synthesis", Third Edition, 1999, John Wiley & Sons, Inc. (In this case, two or more functional groups may be protected with one protecting group.) Further, the "unsaturated group", "aromatic ring group", "aromatic ring oxy group" and "aliphatic heterocyclic group" may be substituted with halogen atoms, azide group, nitro group, lower alkyl groups, lower haloalkyl groups, lower alkoxy groups, lower haloalkoxy groups, lower alkylamino groups, lower alkylthio groups, cyano group, lower alkoxycarbonyl groups, aminocarbonyl group, lower alkylaminocarbonyl groups, hydroxyl group, protected hydroxyl groups, amino group, protected amino groups, thiol group, protected thiol groups, aldehyde group, protected aldehyde groups, carboxyl group or protected carboxyl groups. Although some of these substituent groups may react with sulfuryl fluoride in the presence of the organic base and the nucleophile, the desired reaction can be promoted favorably by adoption of the suitable reaction conditions.

When two of the substituent groups R¹, R² and R³ are any other than hydrogen atom, formyl group, aminocarbonyl group and cyano group in the alcohol of the general formula [1], these two substituent groups may form a ring structure by a covalent bond between two carbon atoms thereof through or without a heteroatom (e.g. nitrogen, oxygen, sulfur etc.).

The carbon atom to which the hydroxyl group is bonded is an asymmetric carbon atom when the substituent groups R¹, R² and R³ are different in kind from one another in the alcohol of the general formula [1]. The configuration of this asymmetric carbon atom is inverted in the reaction. In the case where the target compound is in the form of an optically active substance, an optically active alcohol is used as the raw substrate. (It is needless to say that the raw substrate can be a racemic mixture of the alcohol depending on the target compound.)

In the optically active alcohol of the general formula [3], R⁴ and R⁵ each independently represent an alkyl group, a substituted alkyl group, a formyl group, an alkylcarbonyl group, a substituted alkylcarbonyl group, an arylcarbonyl group, a substituted arylcarbonyl group, an alkoxycarbonyl group, a substituted alkoxycarbonyl group, an aminocarbonyl group, an alkylaminocarbonyl group, a substituted alkylaminocarbonyl group, an arylaminocarbonyl group, a substituted arylaminocarbonyl group, or a cyano group. These substituent groups are R⁴ and R⁵ different in kind from each other. Specific examples of the substituent groups R⁴ and R⁵ are the same as those of R¹, R², R³ in the alcohol of the general formula [1].

When the substituent groups R⁴ and R⁵ are any other than formyl group, aminocarbonyl group and cyano group in the optically active alcohol of the general formula [3], these substituent groups may form a ring structure by a covalent bond between two carbon atoms thereof through or without a heteroatom (e.g. nitrogen, oxygen, sulfur etc.) (whereby the optically active alcohol can be, for example, an optically active hydroxycycloalkane).

In the optically active alcohol of the general formula [3], * represents an asymmetric carbon atom. The configuration of this asymmetric carbon atom is inverted in the reaction.

The asymmetric carbon atom of the optically active alcohol of the general formula [3] can be in a R-configuration and/or S-configuration. The configuration of the asymmetric carbon atom of the optically active alcohol of the general formula [3] can be selected as appropriate depending on the absolute configuration of the target compound.
It suffices that the optical purity of the optically active alcohol of the general formula [3] is 70%ee or higher (enantiomer excess). The optical purity of the optically active alcohol of the general formula [3] is generally preferably 80%ee or higher, more preferably 90%ee or higher.

In the optically active α-hydroxyester of the general formula [5], R⁶ and R⁷ each independently represent an alkyl group or a substituted alkyl group. Specific examples of the substituent groups R⁶ and R⁷ are the same as those of R¹, R², R³ in the alcohol of the general formula [1].

The substituent groups R⁶ and R⁷ may form a ring structure by a covalent bond between two carbon atoms thereof through or without a heteroatom (e.g. nitrogen, oxygen, sulfur etc.) in the optically active α-hydroxyester of the general formula [5] (whereby the optically active α-hydroxyester can be, for example, an optically active α-hydroxylactone).

In the optically active α-hydroxyester of the general formula [5], * represents an asymmetric carbon atom. The configuration of this asymmetric carbon atom is inverted in the reaction.

The asymmetric carbon atom of the optically active α-hydroxyester of the general formula [5] can be in a R-configuration and/or S-configuration. The configuration of the asymmetric carbon atom of the optically active α-hydroxyester of the general formula [5] can be selected as appropriate depending on the absolute configuration of the target compound. It suffices that the optical purity of the optically active α-hydroxyester of the general formula [5] is 80%ee or higher. The optical purity of the optically active α-hydroxyester of the general formula [5] is generally preferably 90%ee or higher, more preferably 95%ee or higher.

As a suitable raw substrate of the present invention, the optically active α-hydroxyester of the general formula [5] can be prepared from various commercially available optically active α-amino acids in the same manner as disclosed in Synthetic Communications (U.S.), 1991, Vo1.21, P.2165-2170 etc. Some forms of the optically active α-hydroxyester are commercially available and usable as the raw substrate. For example, there was used commercially available ethyl ester of (S)-lactic acid in the after-mentioned examples. Further, the alcohol of the general formula [1] and the optically active alcohol of the general formula [3] are commercially available in various forms.

In the optically active 4-hydroxyproline of the general formula [7], R⁸ represents a secondary amino protecting group. Examples of the secondary amino protecting group are benzyloxycarbonyl, tert-butoxycarbonyl, 9-fluorenylmethoxycarbonyl, 3-nitro-2-pyridinesulfenyl and p-methoxybenzyloxycarbonyl.
Among others, benzyloxycarbonyl and tert-butoxycarbonyl are preferred. Particularly preferred is tert-butoxycarbonyl.

In the optically active 4-hydroxyproline of the general formula [7], R⁹ represents a carboxyl protecting group. Examples of the carboxyl protecting group are methyl, ethyl, tert-butyl, trichloroethyl, phenacyl, benzyl, 4-nitrobenzyl and 4-methoxybenzyl. Among others, methyl, ethyl, tert-butyl and benzyl are preferred. Particularly preferred are methyl and ethyl.

As a suitable raw substrate of the present invention, the optically active 4-hydroxyproline of the general formula [7] can be prepared from commercially available optically active 4-hydroxyproline in the same manner as disclosed in Jikken Kagaku Koza, Fourth Edition, Vol. 22, Organic Synthesis IV, Acids, Amino Acids and Peptides (published by Maruzen Co., Ltd., 1992, P.193-309) etc. Some forms of the optically active 4-hydroxyproline are commercially available and usable as the raw substrate depending on the combination of the secondary amino protecting group R⁸ and the carboxyl protecting group R⁹. Further, the optically active 4-hydroxyproline of the general formula [7] can be readily prepared, in compound form where the secondary amino protecting group R⁸ is tert-butoxycarbonyl and the carboxyl protecting group R⁹ is methyl (S-configuration at 2-position, R-configuration at 4-position), from hydrochloride of optically active 4-hydroxyproline methyl ester according to Tetrahedron Letters (UK), 1998, Vol.39, P.1169-1172.

In optically active 4-hydroxyproline of the general formula [7], * each represent an asymmetric carbon atom. The configuration of the asymmetric carbon atom at 2-position is maintained throughout the reaction, whereas the configuration of the asymmetric carbon atom at 4-position is inverted in the reaction.

The configurations of the two asymmetric carbon atoms of the optically active 4-hydroxyproline of the general formula [7] can be selected as appropriate depending on the absolute configuration of the target compound. The following combinations of the configurations of the two asymmetric carbon atoms of the optically active 4-hydroxyproline of the general formula [7] are possible: R-configuration at 2-position/R-configuration at 4-position; R-configuration at 2-position/S-configuration at 4-position; S-configuration at 2-position/R-configuration at 4-position; and S-configuration at 2-position/S-configuration at 4-position. It suffices that the enantiomer excess of the optically active 4-hydroxyproline of the general formula [7] is 80%ee or higher. The enantiomer excess of the optically active 4-hydroxyproline of the general formula [7] is generally preferably 90%ee or higher, more preferably 95%ee or higher. Further, it suffices that the diastereoisomer excess of the optically active 4-hydroxyproline of the general formula [7] is 80%de or higher. The diastereoisomer excess of the optically active 4-hydroxyproline of the general formula [7] is generally preferably 90%de or higher, more preferably 95%de or higher.

Examples of the organic base are trimethylamine, triethylamine, diisopropylethylamine, tri-n-propylamine, tri-n-butylamine, tri-n-pentylamine, tri-n-hexylamine, pyridine, 2,3-lutidine, 2,4-lutidine, 2,5-lutidine, 2,6-lutidine, 3,4-lutidine, 3,5-lutidine, 2,3,4-collidine, 2,4,5-collidine, 2,5,6-collidine, 2,4,6-collidine, 3,4,5-collidine, 3,5,6-collidine, 4-dimethylaminopyridine, 1,5-diazabicyclo[4.3.0]non-5-ene (DBN), 1,8-diazabicyclo[5.4.0]undec-7-ene (DBU), N,N,N',N',N"-pentamethylguanidine, 1,5,7-triazabicyclo[4.4.0]dec-5-ene (TBD) and phosphazene bases e.g. BEMP and t-Bu-P4. Among others, triethylamine, diisopropylethylamine, tri-n-butylamine, pyridine, 2,6-lutidine, 2,4,6-collidine, 4-dimethylaminopyridine, 1,5-diazabicyclo[4.3.0]non-5-ene and 1,8-diazabicyclo[5.4.0]undec-7-ene are preferred. Particularly preferred are triethylamine, diisopropylethylamine, tri-n-butylamine, pyridine, 2,6-lutidine, 2,4,6-collidine and 1,8-diazabicyclo[5.4.0]undec-7-ene. These organic bases can be used solely or in combination thereof.

It suffices to use the organic base in an amount of 0.6 mol or more per 1 mol of the alcohol of the general formula [1]. The amount of the organic base used is preferably 0.7 to 10 mol, more preferably 0.8 to 5 mol, per 1 mol of the alcohol of the general formula [1].

The nucleophile constituting species X, which constitutes the nucleophile (X⁻), is a halogen atom such as chlorine, bromine or iodine, an azide group, a nitrooxy group, a cyano group, a thiocyanate group, a formyloxy group, an alkylcarbonyloxy group, a substituted alkylcarbonyloxy group, an arylcarbonyloxy group or a substituted arylcarbonyloxy group. Among others, a halogen atom such as chlorine, bromine or iodine, an azide group, a formyloxy group, an alkylcarbonyloxy group, a substituted alkylcarbonyloxy group, an arylcarbonyloxy group and a substituted arylcarbonyloxy group are preferred. Particularly preferred are a halogen atom such as chlorine, bromine or iodine and an azide group. Herein, the nitrooxy group refers to a group represented by ONO₂; and the formyloxy group refers to a group represented by OCOH. The alkyl moiety (R) of the alkylcarbonyloxy group (OCOR) and the substituted alkyl moiety (R') of the substituted alkylcarbonyloxy group (OCOR') have the same definitions as those of R¹, R², R³ in the alcohol of the general formula [1]. The aryl moiety (Ar) of the arylcarbonyloxy group (OCOAr) and the substituted aryl moiety of the substituted arylcarbonyloxy group (OCOAr') have the same definitions as those of R¹, R², R³ in the alcohol of the general formula [1].

The nucleophile (X⁻) may be in the form of a salt with a counter cation. Alternatively, the nucleophile (X⁻) may be in the form of a neutral molecule in which the atom X is involved in a covalent bond so that the anion X⁻ is liberated from the neutral molecule and functions as the nucleophile in the reaction system.

Examples of the counter cation (or the covalent bond group (the counter group of the covalent bond) of the neutral molecule) usable in combination with the nucleophile (X⁻) are: monovalent cations of alkali metals such as lithium, sodium, potassium and cesium; divalent cations of alkaline-earth metals such as magnesium and calcium; monovalent or divalent cations of Group-11 (IB) transition metals such as copper and silver; monovalent cations of quaternary ammoniums such as tetramethylammonium, tetraethylammonium and tetrabutylammonium; monovalent cations of quaternary phosphoniums such as tetramethylphosphonium, tetraethylphosphonium and tetrabutylphosphonium; monovalent covalent bond groups of trialkylsilyls such as trimethylsilyl, triethylsilyl and tert-butyldimethylsilyl; and monovalent covalent bonds of phosphoryls such as diphenylphosphoryl. Among others, monovalent cations of alkali metals, divalent cations of alkaline-earth metals, monovalent cations of quaternary ammoniums and monovalent cations of quaternary phosphoniums are preferred. Particularly preferred are monovalent cations of alkali metals, divalent cations of alkaline-earth metals and monovalent cations of quaternary ammoniums. These counter cations (or neutral molecule's covalent bond groups) can be used solely or in combination thereof. As quaternary ammoniums and quaternary phosphoniums are often used as phase transfer catalysts for organic synthesis, the combined use of "an alkali metal salt or alkaline-earth metal salt" and "a quaternary ammonium salt or quaternary phosphonium salt" provides the same effect as in the case where the reaction with the alkali metal salt or alkaline-earth metal salt is conducted in the presence of the phase transfer catalyst. It is thus one preferred embodiment of the present invention to use "alkali metal salt or alkaline-earth metal salt" in combination with "quaternary ammonium salt or quaternary phosphonium salt".

Preferred combinations of the counter cation (neutral molecule's covalent bond group) and the species X constituting the nucleophile (X⁻) are: monovalent alkali metal cation/halogen atom; divalent alkaline-earth metal cation/halogen atoms (two atoms); monovalent quaternary ammonium cation/halogen atom; monovalent quaternary phosphonium cation/halogen atom; monovalent alkali metal cation/azide group; divalent alkaline-earth metal cation/azide groups (two groups); monovalent quaternary ammonium cation/azide group; monovalent quaternary phosphonium cation/azide group; monovalent alkali metal cation/formyloxy group; divalent alkaline-earth metal cation/formyloxy groups (two groups); monovalent quaternary ammonium cation/formyloxy group; monovalent quaternary phosphonium cation/formyloxy group; monovalent alkali metal cation/alkylcarbonyloxy group; divalent alkaline-earth metal cation/alkylcarbonyloxy groups (two groups); monovalent quaternary ammonium cation/alkylcarbonyloxy group; monovalent quaternary phosphonium cation/alkylcarbonyloxy group; monovalent alkali metal cation/substituted alkylcarbonyloxy group; divalent alkaline-earth metal cation/substituted alkylcarbonyloxy groups (two groups); monovalent quaternary ammonium cation/substituted alkylcarbonyloxy group; monovalent quaternary phosphonium cation/substituted alkylcarbonyloxy group; monovalent alkali metal cation/arylcarbonyloxy group; divalent alkaline-earth metal cation/arylcarbonyloxy groups (two groups); monovalent quaternary ammonium cation/arylcarbonyloxy group; monovalent quaternary phosphonium cation/arylcarbonyloxy group; monovalent alkali metal cation/substituted arylcarbonyloxy group; divalent alkaline-earth metal cation/substituted arylcarbonyloxy groups (two groups); monovalent quaternary ammonium cation/substituted arylcarbonyloxy group; and monovalent quaternary phosphonium cation/substituted arylcarbonyloxy group. Particularly preferred are: monovalent alkali metal cation/halogen atom; divalent alkaline-earth metal cation/halogen atoms (two atoms); monovalent quaternary ammonium cation/halogen atom; monovalent alkali metal cation/azide group; divalent alkaline-earth metal cation/azide groups (two groups); and monovalent quaternary ammonium cation/azide group.

As the nucleophile (X⁻), there can also suitably be used a "salt or complex" consisting of "any of hydrogen halides such as hydrogen chloride, hydrogen bromide and hydrogen iodide, hydrogen azide, nitric acid, hydrogen cyanide, thiocyanic acid, formic acid, aliphatic carboxylic acid, substituted aliphatic carboxylic acid, aromatic carboxylic acid and substituted aromatic carboxylic acid (hereinafter referred to as "component A")" and "any of the above-mentioned organic bases usable in the present invention (hereinafter referred to as "component B")". It suffices that the mole ratio of the components A and B of the salt or complex is in the range of 100:1 to 1:100. The mole ratio of the components A and B of the salt or complex is preferably in the range of 50:1 to 1:50, more preferably 25:1 to 1:25. The salt or complex can be readily prepared by mixing the components A and B at a desired ratio with caution given to heat generation. It is particularly convenient to prepare the salt or complex in the after-mentioned reaction solvent and directly use the resulting solution for the reaction. As a matter of course, a commercially available product of this salt or complex is also usable. In view of the fact that, in Scheme 1, sulfonylation favorably proceeds under basic conditions, it is effective to increase the amount of the above-mentioned organic base used and thereby place the reaction system under basic conditions in the case where the mole ratio of the component A is significantly higher than that of the component B.

It suffices to use the nucleophile (X⁻) in an amount of 0.6 mol or more per 1 mol of the alcohol of the general formula [1]. The amount of the nucleophile (X⁻) used is preferably 0.7 to 10 mol, more preferably 0.8 to 5 mol, per 1 mol of the alcohol of the general formula [1].

Further, it suffices to use the sulfuryl fluoride in an amount of 0.7 mol or more per 1 mol of the alcohol of the general formula [1]. The amount of the sulfuryl fluoride used is 0.8 to 10 mol, more preferably 0.9 to 5 mol, per 1 mol of the alcohol of the general formula [1].

Examples of the reaction solvent are: aliphatic hydrocarbon solvents such as n-hexane, cyclohexane and n-heptane; aromatic hydrocarbon solvents such as benzene, toluene, ethylbenzene, xylene and mesitylene; halogenated hydrocarbon solvents such as methylene chloride, chloroform and 1,2-dichloroethane; ether solvents such as diethyl ether, tetrahydrofuran, diisopropyl ether and tert-butyl methyl ether, ester solvents such as ethyl acetate and n-butyl acetate; amide solvents such as N,N-dimethylformamide, N,N-dimethylacetamide, N-methylpyrrolidone, 1,3-dimethyl-2-imidazolidinone; nitrile solvents such as acetonitrile and propionitrile; and dimethyl sulfoxide. Among others, n-hexane, n-heptane, toluene, xylene, mesitylene, methylene chloride, tetrahydrofuran, diisopropyl ether, tert-butyl methyl ether, ethyl acetate, N,N-dimethylformamide, N,N-dimethylacetamide, acetonitrile, propionitrile and dimethyl sulfoxide are preferred. Particularly preferred are toluene, xylene, methylene chloride, tetrahydrofuran, tert-butyl methyl ether, ethyl acetate, N,N-dimethylformamide and acetonitrile. These reaction solvents can be used solely or in combination thereof.

It suffices to use the reaction solvent in an amount of 0.05 L or more per 1 mol of the alcohol of the general formula [1]. The amount of the reaction solvent used is 0.1 to 20 L, more preferably 0.2 to 10 L, per 1 mol of the alcohol of the general formula [1].

It suffices that the reaction temperature is in the range of -60 to +100°C. The reaction temperature is preferably -40 to +80°C, more preferably -20 to +60°C.

Further, it suffices that the reaction time is 48 hours or less. As the reaction time depends on the raw substrate and the reaction conditions, it is preferable to determine the time at which the raw substrate has almost disappeared as the end of the reaction while monitoring the progress of the reaction by any analytical means such as gas chromatography, liquid chromatography or nuclear magnetic resonance.

The hydroxyl group substitution group of the general formula [2] can be obtained as a crude product by post treatment of the reaction terminated liquid. As one example of post treatment operation, it is feasible to concentrate the reaction terminated liquid as appropriate, dilute the concentrated liquid with organic solvent (such as n-hexane, n-heptane, toluene, xylene, methylene chloride, diisopropyl ether, tert-butyl methyl ether, ethyl acetate etc.), wash the diluted liquid with water, aqueous solution of inorganic acid (such as hydrochloric acid, hydrobromic acid, hydroiodic acid, nitric acid, sulfuric acid etc.) or aqueous solution of inorganic base (such as sodium hydrogencarbonate, potassium hydrogencarbonate, sodium carbonate, potassium carbonate, sodium hydroxide, potassium hydroxide etc.), (dry the recovered organic layer with a drying agent such as anhydrous sodium sulfate, anhydrous magnesium sulfate etc. as needed,) and then, concentrate the recovered organic layer. There may be some quaternary ammonium salt or quaternary phosphonium salt remaining in the crude product. In this case, it is feasible to extract the salt and the target compound with an organic solvent against which the salt and the target compound show a difference in solubility (such as n-hexane, n-heptane, toluene, xylene etc.) so that the target compound and the salt can be readily separated by simple operation such as filtration. It is also effective to use a short column of silica or alumina for removal of the salt. Further, the crude product can be purified to a high chemical purity, as required, by purification operation such as activated carbon treatment, distillation, recrystallization or column chromatography. When the target hydroxyl group substitution product is unstable, it is feasible to subject the recovered organic layer directly to the subsequent reaction step.

As mentioned above, it is possible in the present invention that the hydroxyl group substitution product can be manufactured by reaction of the alcohol with sulfuryl fluoride in the presence of the organic base and nucleophile (Inventive Aspect 1).

Among Inventive Aspect 1, the following material combination is preferred (Inventive Aspect 2): the raw substrate is an optically active alcohol in which one of three substituent groups is a hydrogen atom and the other two of three substituent groups are different in kind from each other and are each independently selected from an alkyl group, a substituted alkyl group, a formyl group, an alkylcarbonyl group, a substituted alkylcarbonyl group, an arylcarbonyl group, a substituted arylcarbonyl group, an alkoxycarbonyl group, a substituted alkoxycarbonyl group, an aminocarbonyl group, an alkylaminocarbonyl group, a substituted alkylaminocarbonyl group, an arylaminocarbonyl group, a substituted arylaminocarbonyl group and a cyano group; and the nucleophile constituting species X is a halogen atom such as chlorine, bromine or iodine, an azide group, a formyloxy group, an alkylcarbonyloxy group, a substituted alkylcarbonyloxy group, an arylcarbonyloxy group or a substituted arylcarbonyloxy group. The combination of the raw substrate and the nucleophile according to this aspect is advantageous in that: the desired reaction proceeds very favorably; and the obtained optically active hydroxyl group substitution product is very important as an intermediate for pharmaceutical and agricultural chemicals.

The following material combination is particularly preferred (Inventive Aspect 3) among Inventive Aspect 2: the raw substrate is an optically active α-hydroxyester in which one of three subsitutent groups is a hydrogen atom, another one of three substituent groups is an alkoxycarbonyl group or a substituted alkoxycarbonyl group and the other one of three substituent groups is an alkyl group or a substituted alkyl group; and the nucleophile constituting species X is a halogen atom such as chlorine, bromine or iodine or an azide group. The combination of the raw substrate and the nucleophile according to this aspect is advantageous in that: the desired reaction proceeds extremely favorably; and the obtained optically active α-hydroxyl group substitution ester is extremely important as an intermediate for pharmaceutical and agricultural chemicals.

The following material combination is also particularly preferred (Inventive Aspect 4) among Inventive Aspect 2: the raw substrate is an optically active 4-hydroxyproline in which a secondary amino group and an carboxyl group are protected by protecting groups, respectively; and the nucleophile constituting species X is a halogen atom such as chlorine, bromine or iodine or an azide group. The combination of the raw substrate and the nucleophile according to this aspect is also advantageous in that: the desired reaction proceeds extremely favorably; and the obtained optically active 4-hydroxyl group substitution proline is extremely important as an intermediate for pharmaceutical and agricultural chemicals.

### Examples

The present invention will be described in more detail below by way of the following examples. It should be noted that these examples are illustrative and are not intended to limit the present invention thereto. In the following description, the abbreviations "Me", "Et", "Boc" and "Ac" refer to methyl, ethyl, tert-butoxycarbonyl and acetyl, respectively.

### [Example 1]

Into a pressure-proof reaction vessel of stainless steel (SUS) were placed 4.73 g (40.0 mmol, 1.00 eq) of optically active α-hydroxyester of the following formula (S-configuration, 98%ee or higher): 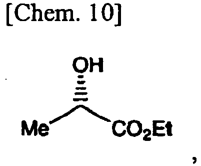 40 mL (1.00 M) of acetonitrile, 4.65 g (36.0 mmol, 0.90 eq) of diisopropylethylamine and 11.6 g (36.0 mmol, 0.90 eq) of tetrabutylammonium bromide. Then, 8.16 g (80.0 mmol, 2.00 eq) of sulfuryl fluoride was blown from a cylinder into the reaction vessel under ice cooling conditions. The resulting reaction mixture solution was stirred for 2 hours and 50 minutes under ice cooling conditions. A part of the reaction mixture solution was diluted with ethyl acetate, washed with water, and then, analyzed by gas chromatography.

It was confirmed by the analytical results that: the conversion rate was 81%; the area percentage of optically active α-hydroxyl group substitution ester of the following formula (R-configuration): 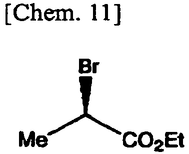 was 73.8%; and the area percentage of fluorinated compound of the following formula: 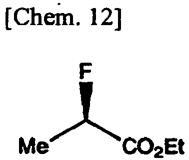 was 2.3%.

The area percentage ratio of the optically active α-hydroxyl group substitution ester and the fluorinated compound was 97:3. The optical purity of the optically active α-hydroxyl group substitution ester (R-configuration) was 91.8%ee. The ¹H-NMR data of the optically active α-hydroxyl group substitution ester are indicated below. (There was almost no quaternary ammonium salt contained.)

¹H-NMR [reference material: (CH₃)₄Si, deuterium solvent: CDCl₃] δ ppm; 1.30 (t, 7.2Hz, 3H), 1.83 (d, 6.8Hz, 3H), 4.23 (q, 7.2Hz, 2H), 4.36 (q, 6.8Hz, 1H).

### [Example 2]

Into a pressure-proof reaction vessel of stainless steel (SUS) were placed 4.73 g (40.0 mmol, 1.00 eq) of optically active α-hydroxyester of the following formula (S-configuration, 98%ee or higher): 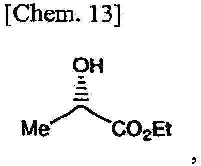 40 mL (1.00 M) of acetonitrile, 4.86 g (48.0 mmol, 1.20 eq) of triethylamine, 6.16 g (40.0 mmol, 1.00 eq) of tetramethylammonium bromide and 13.3 g (41.3 mmol, 1.03 eq) of tetrabutylammonium bromide. Then, 8.16 g (80.0 mmol, 2.00 eq) of sulfuryl fluoride was blown from a cylinder into the reaction vessel under ice cooling conditions. The resulting reaction mixture solution was stirred for 2 hours under ice cooling conditions. A part of the reaction mixture solution was diluted with ethyl acetate, passed through a short column of silica (for removal of origin component), and then, analyzed by gas chromatography.

It confirmed by the analytical results that: the conversion rate of the reaction was 100%; the area percentage of optically active α-hydroxyl group substitution ester of the following formula (R-configuration): 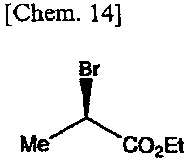 was 94.5%; and the area percentage of fluorinated compound of the following formula: 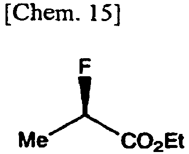 was 1.8%. The area percentage ratio of the optically active α-hydroxyl group substitution ester and the fluorinated compound was 98:2. The optical purity of the optically active α-hydroxyl group substitution ester (R-configuration) was 88.3%ee.

The ¹H-NMR data of the optically active α-hydroxyl group substitution ester was the same as that of Example 1. (There was almost no quaternary ammonium salt contained.)

### [Example 3]

Into a pressure-proof reaction vessel of stainless steel (SUS) were placed 5.00 g (20.4 mmol, 1.00 eq) of optically active 4-hydroxyproline of the following formula (S-configuration at 2-position/R-configuration at 4-position, 98%ee or higher, 98%de or higher): 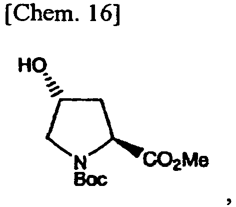 20 mL (1.02 M) of acetonitrile, 4.13 g (40.8 mmol, 2.00 eq) of triethylamine and 13.1 g (40.6 mmol, 1.99 eq) of tetrabutylammonium bromide. Then, 4.16 g (40.8 mmol, 2.00 eq) of sulfuryl fluoride was blown from a cylinder into the reaction vessel under ice cooling conditions. The resulting reaction mixture solution was stirred for 2 hours and 30 minutes under ice cooling conditions. It was confirmed by ¹H-NMR analysis of the reaction mixture solution that the conversion rate of the reaction was 100%.

The thus-obtained reaction terminated liquid was concentrated under a reduced pressure to about one-third volume, diluted with 100 mL of toluene and washed six times with 50 mL of water. The organic layer was recovered, concentrated under a reduced pressure and subjected to vacuum drying, thereby yielding 6.40 g of a crude product of optically active 4-hydroxyl group substitution proline of the following formula (S-configuration at 2-position/S-configuration at 4-position): 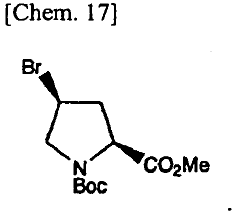 The yield of the product was quantitative.

It was confirmed by ¹H-NMR and ¹⁹F-NMR analysis of the crude product that no fluorinated compound of the following formula: 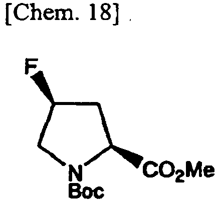 was contained (less than 3 mol%). The ¹H-NMR data of the optically active 4-hydroxyl group substitution proline are indicated below. (There was almost no quaternary ammonium salt contained.)

¹H-NMR [reference material: (CH₃)₄Si, deuterium solvent: CDCl₃] δ ppm; 1.42 (s, part of 9H), 1.47 (s, part of 9H), 2.42 (m, 1H), 2.84 (m, 1H), 3.73 (m, 1H), 3.77 (s, 3H), 4.06 (m, 1H), 4.20-4.50 (m, 2H).

The optically active 4-hydroxyl group substitution proline of the above formula can be converted, through an azide compound of the following formula (S-configuration at 2-position/R-configuration at 4-position): 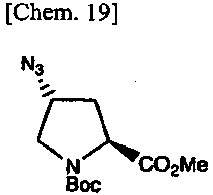 to an amino compound of the following formula (S-configuration at 2-position/R-configuration at 4-position): 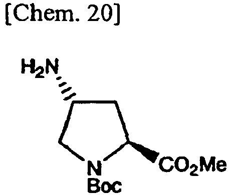 with reference to Patent Document 1, Journal of Medicinal Chemistry (U.S.), 2006, Vol.49, P.307-317 and the like.

### [Example 4]

Into a pressure-proof reaction vessel of stainless steel (SUS) were placed 49.1 g (200 mmol, 1.00 eq) of optically active 4-hydroxyproline of the following formula (S-configuration at 2-position/R-configuration at 4-position, 98%ee or higher, 98%de or higher): 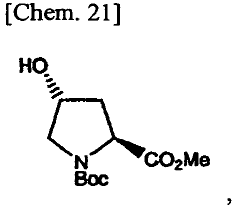 195 mL (1.03 M) of toluene, 200 mL (1.00 M) of acetonitrile, 40.5 g (400 mmol, 2.00 eq) of triethylamine and 133 g (413 mmol, 2.07 eq) of tetrabutylammonium bromide. Then, 40.8 g (400 mmol, 2.00 eq) of sulfuryl fluoride was blown from a cylinder into the reaction vessel under salt-ice cooling conditions. The resulting reaction mixture solution was stirred for 2 hours under salt-ice cooling conditions. It was confirmed by ¹H-NMR analysis of the reaction mixture solution that the conversion rate of the reaction was 100%.

The thus-obtained reaction terminated liquid was washed with 300 mL of water. The organic layer was recovered, concentrated under a reduced pressure and subjected to vacuum drying, thereby yielding 145 g of a crude product of optically active 4-hydroxyl group substitution proline of the following formula (S-configuration at 2-position/S-configuration at 4-position):

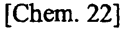

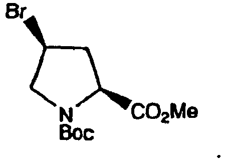

It was confirmed by ¹H-NMR and ¹⁹F-NMR analysis of the crude product that no fluorinated compound of the following formula: 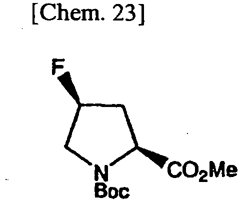 was contained (less than 3 mol%).

It was also confirmed that there was a considerable amount of quaternary ammonium salt contained in the crude product (the mole ratio of the target compound and the quaternary ammonium salt was 53:47). To 68.4 g (estimated as 94.3 mmol) of the crude product, 51 mL (1.85 M) of toluene and 120 mL (0.786 M) of n-heptane were added. The resulting solution was stirred for 2 hours at room temperature, followed by filtering crystalline matter (quaternary ammonium salt) out of the solution. The filtration residue was washed with a small amount of n-heptane. The thus-obtained filtrate was concentrated under a reduced pressure and subjected to vacuum drying, thereby recovering 28.4 g of a purified product of optically active 4-hydroxyl group substitution proline of the above formula. It was confirmed by ¹H-NMR analysis of the purified product that the quaternary ammonium salt had been totally removed (less than 3 mol%). The yield of the product was 98%. The ¹H-NMR data of the optically active 4-hydroxyl group substitution proline was the same as that of Example 3.

To 11.8 g (38.3 mmol, 1.00 eq) of the purified product of the optically active 4-hydroxyl group substitution proline of the above formula, 77 mL (0.497 M) of N,N-dimethylformamide and 2.74 g (42.1 mmol, 1.10 eq) of sodium azide were added. The resulting reaction mixture solution was stirred for 2 days at room temperature. It was confirmed by ¹H-NMR analysis of the reaction mixture solution that the conversion rate of the reaction was 100%.

The thus-obtained reaction terminated liquid was diluted with 200 mL of ethyl acetate and washed three times with 100 mL of water. The organic layer was recovered, concentrated under a reduced pressure and subjected to vacuum drying, thereby yielding 12.5 g of a crude product of an azide compound of the following formula (S-configuration at 2-position/R-configuration at 4-position): 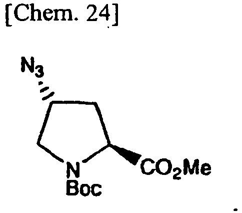 It was confirmed by ¹H-NMR analysis (quantitative analysis) of the crude product that 9.16 g of the target compound was contained (there was a considerable amount of N,N-dimethylformamide contained). The yield of the product was 89%. The ¹H-NMR data of the azide compound are indicated below. (No 4-position epimer (S-configuration at 4-position) was contained (less than 3 mol%).)

¹H-NMR [reference material: (CH₃)₄Si, deuterium solvent: CDCl₃] δ ppm; 1.42 (s, part of 9H), 1.47 (s, part of 9H), 2.18 (m, 1H), 2.33 (m, 1H), 3.42-3.84 (m, 2H), 3.74 (s, 3H), 4.20 (m, 1H), 4.38 (m, 1H).

To 12.5 g (estimated as 33.9 mmol, 1.00 eq) of the crude product of the azide compound of the above formula, 34 mL (0.997 M) of methanol and 2.89 g (50% water content, 0.679 mmol, 0.02 eq) of 5% palladium/activated carbon were added. The resulting reaction mixture solution was stirred for one night at room temperature while setting the pressure of hydrogen gas (H₂) at 0.15 MPa. It was confirmed by ¹H-NMR analysis of the reaction mixture solution that the conversion rate of the reaction was 100%.

The thus-obtained reaction terminated liquid was subjected to Celite filtration. The filtrate was admixed with 3.53 g (33.9 mmol, 1.00 eq) of 35% hydrochloric acid, stirred for 15 minutes at room temperature, concentrated under a reduced pressure, subjected to azeotropic dehydration twice with 100 mL of toluene, and then, subjected to vacuum drying. With this, 11.1 g of a crude product of hydrochloride of amino compound of the following formula (S-configuration at 2-position/R-configuration at 4-position): 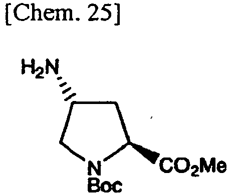 was yielded (There was contained a small amount of N,N-dimethylformamide and toluene). The yield of the product was quantitative. To the whole (estimated as 33.9 mmol) of the crude product of the hydrochloride, 30 mL (1.13 M) of ethyl acetate, 30 mL (1.13 M) of n-hexane and 6 mL (5.65 M) of isopropanol were added. The crude product of the hydrochloride was dissolved into the solvent by heating. The resulting solution was cooled down to room temperature, followed by filtering crystalline precipitate out of the solution. The filtration residue was washed with a small amount of n-hexane and subjected to vacuum drying, thereby recovering 3.95 g of a purified product of hydrochloride of amino compound of the above formula.

The gas chromatographic purity of the purified product (free base) was 99.2%. The yield of the product until recrystallization was 41 %. (The recrystallization conditions were not optimized.) The ¹H-NMR data of the hydrochloride and free base of the amino compound are indicated below.

Hydrochloride: ¹H-NMR [reference material: (CH₃)₄Si, deuterium solvent: CD₃OD] δ ppm; 1.42 (s, part of 9H), 1.47 (s, part of 9H), 2.39 (m, 2H), 3.54 (m, 1H), 3.75 (s, part of 3H), 3.76 (s, part of 3H), 3.80 (m, 1H), 3.93 (m, 1H), 4.45 (m, 1H). (The attributions of the NH₂ and HCl proton peaks were not identifiable.)

Free base: ¹H-NMR [reference material: (CH₃)₄Si, deuterium solvent: CDCl₃] δ ppm; 1.41 (s, part of 9H), 1.46 (s, part of 9H), 1.91-2.19 (m, 2H), 3.06-3.24 (m, 1H), 3.65-3.76 (m, 2H), 3.73 (s, 3H), 4.39 (m, 1H). (The attribution of the NH₂ proton peak was not identifiable.)

### [Example 5]

Into a pressure-proof reaction vessel of stainless steel (SUS) were placed 736 mg (3.00 mmol, 1.00 eq) of optically active 4-hydroxyproline of the following formula (S-configuration at 2-position/R-configuration at 4-position, 98%ee or higher, 98%de or higher): 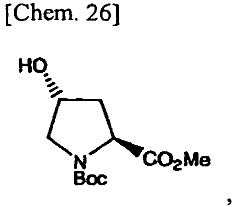 3 mL (1.00 M) of acetonitrile, 610 mg (6.03 mmol, 2.01 eq) of triethylamine and 1.71 g (6.01 mmol, 2.00 eq) of tetrabutylammonium azide. Then, 610 mg (5.98 mmol, 1.99 eq) of sulfuryl fluoride was blown from a cylinder into the reaction vessel under ice cooling conditions. The resulting reaction mixture solution was stirred for 2 hours and 45 minutes under ice cooling conditions. It was confirmed by ¹H-NMR analysis of the reaction mixture solution that the conversion rate of the reaction was 100%.

The thus-obtained reaction terminated liquid was diluted with ethyl acetate, washed five times with saturated sodium chloride solution. The organic layer was recovered, concentrated under a reduced pressure and subjected to vacuum drying. The residue was treated with a short column of silica (ethyl acetate:n-hexane = 1: 1), thereby yielding 600 mg of a crude product of optically active 4-hydroxyl group substitution proline of the following formula (S-configuration at 2-position/S-configuration at 4-position):

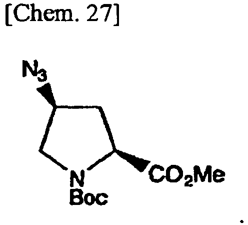

It was confirmed by ¹H-NMR and ¹⁹F-NMR analysis of the crude product that fluorinated compound of the following formula: 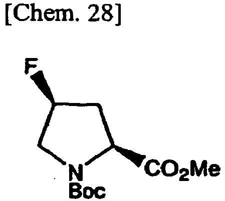 was contained. The mole ratio of the optically active 4-hydroxyl group substitution proline and the fluorinated compound was 57:43. The yield of the optically active 4-hydroxyl group substitution proline was thus 44%. It was also confirmed that no 4-position diastereomer was contained in the optically active 4-hydroxyl group substitution proline.

### [Example 6]

Into a pressure-proof reaction vessel of stainless steel (SUS) were placed 736 mg (3.00 mmol, 1.00 eq) of optically active 4-hydroxyproline of the following formula (S-configuration at 2-position/R-configuration at 4-position, 98%ee or higher, 98%de or higher): 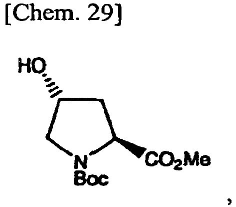 3 mL (1.00 M) of acetonitrile, 610 mg (6.03 mmol, 2.01 eq) of triethylamine, 1.71 g (6.01 mmol, 2.00 eq) of tetrabutylammonium azide and 390 mg (6.00 mmol, 2.00 eq) of sodium azide. Then, 610 mg (5.98 mmol, 1.99 eq) of sulfuryl fluoride was blown from a cylinder into the reaction vessel under ice cooling conditions. The resulting reaction mixture solution was stirred for 1 hour under ice cooling conditions. It was confirmed by ¹H-NMR analysis of the reaction mixture solution that the conversion rate of the reaction was 100%.

Further, it was confirmed by gas chromatography of the reaction mixture solution that the area percentage ratio of the optically active 4-hydroxyl group substitution proline of the following formula (S-configuration at 2-position/S-configuration at 4-position): 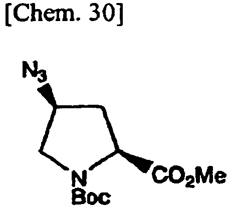 and the fluorinated compound of the following formula: 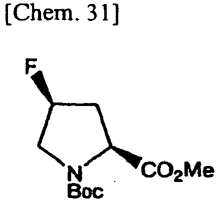 was 84:16. It was also confirmed that no 4-position diastereomer was contained in the optically active 4-hydroxyl group substitution proline.

### [Example 7]

Into a pressure-proof reaction vessel of stainless steel (SUS) were placed 736 mg (3.00 mmol, 1.00 eq) of optically active 4-hydroxyproline of the following formula (S-configuration at 2-position/R-configuration at 4-position, 98%ee or higher, 98%de or higher): 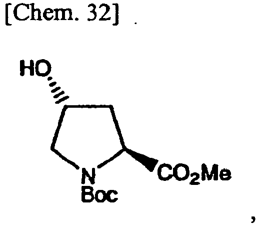 3 mL (1.00 M) of acetonitrile, 610 mg (6.03 mmol, 2.01 eq) of triethylamine and 1.80 g (5.99 mmol, 2.00 eq) of tetrabutylammonium thiocyanate. Then, 610 mg (5.98 mmol, 1.99 eq) of sulfuryl fluoride was blown from a cylinder into the reaction vessel under ice cooling conditions. The resulting reaction mixture solution was stirred for 2 hours and 40 minutes under ice cooling conditions. It was confirmed by ¹H-NMR analysis of the reaction mixture solution that the conversion rate of the reaction was 100%.

Further, it was confirmed by gas chromatography of the reaction mixture solution that the area percentage ratio of the optically active 4-hydroxyl group substitution proline of the following formula: 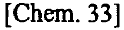 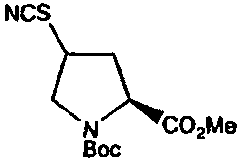 and the fluorinated compound of the following formula: 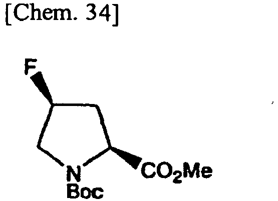 was 70:30. It was also confirmed that there was 4-position diastereomer contained in the optically active 4-hydroxyl group substitution proline. The diastereomer ratio (S-configuration at 2-position/S-configuration at 4-position (estimate): S-configuration at 2-position/R-configuration at 4-position (estimate)) was 79:21.

### [Example 8]

Into a pressure-proof reaction vessel of stainless steel (SUS) were placed 736 mg (3.00 mmol, 1.00 eq) of optically active 4-hydroxyproline of the following formula (S-configuration at 2-position/R-configuration at 4-position, 98%ee or higher, 98%de or higher): 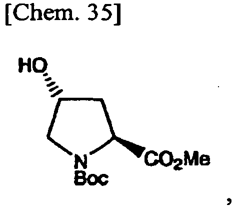 3 mL (1.00 M) of acetonitrile, 610 mg (6.03 mmol, 2.01 eq) of triethylamine and 1.81 g (6.00 mmol, 2.00 eq) oftetrabutylammonium acetate. Then, 610 mg (5.98 mmol, 1.99 eq) of sulfuryl fluoride was blown from a cylinder into the reaction vessel under ice cooling conditions. The resulting reaction mixture solution was stirred for 2 hours and 30 minutes under ice cooling conditions. It was confirmed by ¹H-NMR analysis of the reaction mixture solution that the conversion rate of the reaction was 100%.

The thus-obtained reaction terminated liquid was diluted with ethyl acetate and washed five times with water. The organic layer was recovered, concentrated under a reduced pressure and subjected to vacuum drying. The residue was extracted by stirring with n-hexane (for filtration separation of solid tetrabutylammonium salt), thereby yielding 572 mg of a crude product of optically active 4-hydroxyl group substitution proline of the following formula: 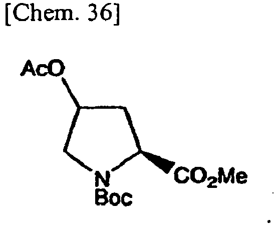 It was confirmed by gas chromatography of the crude product that fluorinated compound of the following formula: 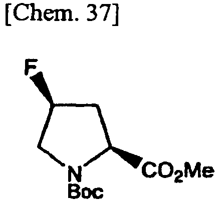 was contained. The area percentage ratio of the optically active 4-hydroxyl group substitution proline and the fluorinated compound was 98:2.

Thus, the yield of the optically active 4-hydroxyl group substitution proline was 65% on the assumption that the area percentage ratio corresponded to the mole ratio. It was also confirmed that there was 4-position diastereomer contained in the optically active 4-hydroxyl group substitution proline. The diastereomer ratio (S-configuration at 2-position/S-configuration at 4-position:S-configuration at 2-position/R-configuration at 4-position) was 72:28.

## Claims

1. A method for manufacturing a hydroxyl group substitution product of the general formula [2], comprising: reacting an alcohol of the general formula [1] with sulfuryl fluoride (SO₂F₂) in the presence of an organic base and a nucleophile (X⁻) 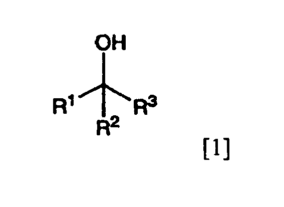 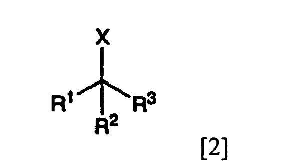 where R¹, R² and R³ each independently represent a hydrogen atom, an alkyl group, a substituted alkyl group, an alkenyl group, a substituted alkenyl group, an alkynyl group, a substituted alkynyl group, an aromatic ring group, a substituted aromatic ring group, a formyl group, an alkylcarbonyl group, a substituted alkylcarbonyl group, an arylcarbonyl group, a substituted arylcarbonyl group, an alkoxycarbonyl group, a substituted alkoxycarbonyl group, an aminocarbonyl group, an alkylaminocarbonyl group, a substituted alkylaminocarbonyl group, an arylaminocarbonyl group, a substituted arylaminocarbonyl group or a cyano group; when two of R¹, R² and R³ are any substituent groups other than hydrogen atom, formyl group, aminocarbonyl group and cyano group, these two substituent groups may form a ring structure by a covalent bond between carbon atoms thereof through or without a heteroatom; and X represents a halogen atom selected from the group consisting of chlorine, bromine and iodine, an azide group, a nitrooxy group, a cyano group, a thiocyanate group, a formyloxy group, an alkylcarbonyloxy group, a substituted alkylcarbonyloxy group, an arylcarbonyloxy group or a substituted arylcarbonyloxy group, wherein, if any of R¹, R² and R³ is a substituted alkyl group, a substituted alkenyl group, a substituted alkynyl group, a substituted aromatic ring group, a a substituted alkylcarbonyl group, a substituted arylcarbonyl group, a substituted alkoxycarbonyl group, a substituted alkylaminocarbonyl group or a substituted arylaminocarbonyl group or if X is a substituted alkylcarbonyloxy group any of the carbon atoms may be substituted, and the substituent is selected from the group consisting of halogen atoms; an azide group; a nitro group; lower alkyl groups; lower haloalkyl groups; lower alkoxy groups; lower haloalkoxy groups; lower alkylamino groups; lower alkylthio groups; a cyano group; lower alkoxycarbonyl groups; an aminocarbonyl group; lower alkylaminocarbonyl groups; lower alkenyl groups; lower alkynyl groups; aromatic ring groups; aromatic ring oxy groups; aliphatic heterocyclic groups; protected hydroxyl groups; protected amino groups; protected thiol groups; an aldehyde group; protected aldehyde groups; protected carboxyl groups and combinations thereof, wherein the term "lower" means that the group to which the term is attached has 1 to 6 carbon atoms in the form of a linear structure, a branched structure or a cyclic structure in the case of 3 or more carbon atoms.

2. The method for manufacturing the hydroxyl group substitution product according to claim 1, wherein an optically active hydroxyl group substitution product of the general formula [4] is manufactured as the hydroxyl group substitution product by reacting an optically active alcohol of the general formula [3] with sulfuryl fluoride (SO₂F₂) in the presence of an organic base and a nucleophile (Y⁻), 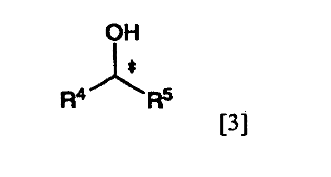 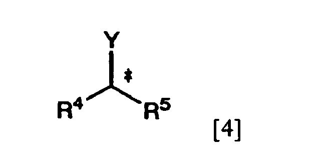 where R⁴ and R⁵ each independently represent an alkyl group, a substituted alkyl group, a formyl group, an alkylcarbonyl group, a substituted alkylcarbonyl group, an arylcarbonyl group, a substituted arylcarbonyl group, an alkoxycarbonyl group, a substituted alkoxycarbonyl group, an aminocarbonyl group, an alkylaminocarbonyl group, a substituted alkylaminocarbonyl group, an arylaminocarbonyl group, a substituted arylaminocarbonyl group, or a cyano group; R⁴ and R⁵ are different in kind from each other; when R⁴ and R⁵ are any substituent groups other than formyl group, aminocarbonyl group and cyano group, the substituent groups may form a ring structure by a covalent bond between carbon atoms thereof through or without a heteroatom; Y represents a halogen atom selected from the group consisting of chlorine, bromine and iodine, an azide group, a formyloxy group, an alkylcarbonyloxy group, a substituted alkylcarbonyloxy group, an arylcarbonyloxy group or a substituted arylcarbonyloxy group; * represents an asymmetric carbon atom; and the configuration of the asymmetric carbon atom is inverted in the reaction, wherein, if any of R⁴ and R⁵ is a substituted alkyl group, a substituted alkylcarbonyl group, a substituted arylcarbonyl group, a substituted alkoxycarbonyl group, a substituted alkylaminocarbonyl group or a substituted arylaminocarbonyl group or ifY is a substituted alkylcarbonyloxy group or a substituted arylcarbonyloxy group any of the carbon atoms may be substituted, and the substituent is selected from the group consisting of halogen atoms; an azide group; a nitro group; lower alkyl groups; lower haloalkyl groups; lower alkoxy groups; lower haloalkoxy groups; lower alkylamino groups; lower alkylthio groups; a cyano group; lower alkoxycarbonyl groups; an aminocarbonyl group; lower alkylaminocarbonyl groups; lower alkenyl groups; lower alkynyl groups; aromatic ring groups; aromatic ring oxy groups; aliphatic heterocyclic groups; protected hydroxyl groups; protected amino groups; protected thiol groups; an aldehyde group; protected aldehyde groups; protected carboxyl groups and combinations thereof, wherein the term "lower" means that the group to which the term is attached has 1 to 6 carbon atoms in the form of a linear structure, a branched structure or a cyclic structure in the case of 3 or more carbon atoms.

3. The method for manufacturing the hydroxyl group substitution product according to claim 2, wherein an optically active α-hydroxyl group substitution ester of the general formula [6] is manufactured as the optically active hydroxyl group substitution product by reacting an optically active α-hydroxyester of the general formula [5] with sulfuryl fluoride (SO₂F₂) in the presence of an organic base and a nucleophile (Z⁻), 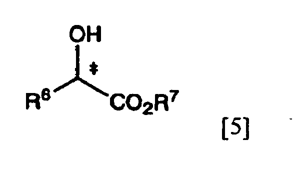 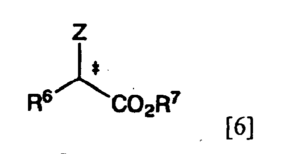 where R⁶ and R⁷ each independently represent an alkyl group or a substituted alkyl group and may form a ring structure by a covalent bond between carbon atoms thereof through or without a heteroatom; Z represents a halogen atom selected from the group consisting of chlorine, bromine and iodine or an azide group; * represents an asymmetric carbon atom; and the configuration of the asymmetric carbon atom is inverted in the reaction, wherein, if any of R⁶ and R⁷ is a substituted alkyl group, the substituent is selected from the group consisting of halogen atoms; an azide group; a nitro group; lower alkyl groups; lower haloalkyl groups; lower alkoxy groups; lower haloalkoxy groups; lower alkylamino groups; lower alkylthio groups; a cyano group; lower alkoxycarbonyl groups; an aminocarbonyl group; lower alkylaminocarbonyl groups; lower alkenyl groups; lower alkynyl groups; aromatic ring groups; aromatic ring oxy groups; aliphatic heterocyclic groups; protected hydroxyl groups; protected amino groups; protected thiol groups; an aldehyde group; protected aldehyde groups; protected carboxyl groups and combinations thereof, wherein the term "lower" means that the group to which the term is attached has 1 to 6 carbon atoms in the form of a linear structure, a branched structure or a cyclic structure in the case of 3 or more carbon atoms.

4. The method for manufacturing the hydroxyl group substitution product according to claim 2, wherein an optically active 4-hydroxyl group substitution proline of the general formula [8] is manufactured as the optically active hydroxyl group substitution product by reacting an optically active 4-hydroxyproline of the general formula [7] with sulfuryl fluoride (SO₂F₂) in the presence of an organic base and a nucleophile (Z⁻), 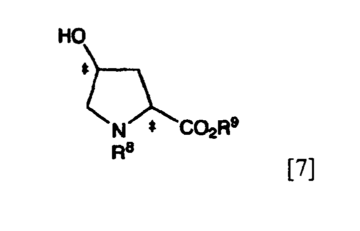 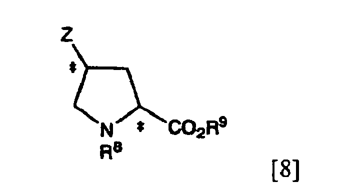 where R⁸ represents a secondary amino protecting group; R⁹ represents a carboxyl protecting group; Z represents a halogen atom selected from the group consisting of chlorine, bromine and iodine or an azide group; and * each represent an asymmetric carbon atom; the configuration of the asymmetric carbon atom at 2-position is maintained throughout the reaction; and the configuration of the asymmetric carbon atom at 4-position is inverted in the reaction.

5. The method for manufacturing the hydroxyl group substitution product according to any of claims 1 to 3, wherein the substituent is an amino acid group or a preptide group as protected amino group.

## Patentansprüche

1. Verfahren zum Herstellen eines Produktes einer Hydroxylgruppensubstitution der allgemeinen Formel [2], das umfasst: Reagieren eines Alkohols der allgemeinen Formel [1] mit Sulfurylfluorid (SO₂F₂) in der Gegenwart einer organischen Base und eines Nucleophils (X⁻) 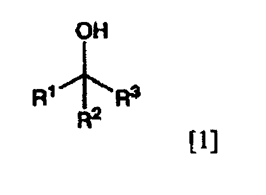 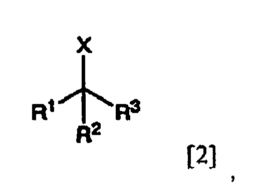 wobei R¹, R² und R³ jeweils unabhängig ein Wasserstoffatom, eine Alkylgruppe, eine substituierte Alkylgruppe, eine Alkenylgruppe, eine substituierte Alkenylgruppe, eine Alkinylgruppe, eine substituierte Alkinylgruppe, eine aromatische Ringgruppe, eine substituierte aromatische Ringgruppe, eine Formylgruppe, eine Alkylcarbonylgruppe, eine substituierte Alkylcarbonylgruppe, eine Arylcarbonylgruppe, eine substituierte Arylcarbonylgruppe, eine Alkoxycarbonylgruppe, eine substituierte Alkoxycarbonylgruppe, eine Aminocarbonylgruppe, eine Alkylaminocarbonylgruppe, eine substituierte Alkylaminocarbonylgruppe, eine Arylaminocarbonylgruppe, eine substituierte Arylaminocarbonylgruppe oder eine Cyanogruppe darstellen; wenn zwei von R¹, R² und R³ beliebige Substituentengruppen mit Ausnahme eines Wasserstoffatoms, Formylgruppe, Aminocarbonylgruppe und Cyanogruppe sind, diese zwei Substituentengruppen eine Ringstruktur durch eine kovalente Bindung zwischen Kohlenstoffatomen davon durch oder ohne ein Heteroatom bilden können; und X ein Halogenatom, das aus der Gruppe ausgewählt wird, die aus Chlor, Brom und lod besteht, eine Azidgruppe, eine Nitrooxygruppe, eine Cyanogruppe, eine Thiocyanatgruppe, eine Formyloxygruppe, eine Alkylcarbonyloxygruppe, eine substituierte Alkylcarbonyloxygruppe, eine Arylcarbonyloxygruppe oder eine substituierte Arylcarbonyloxygruppe darstellt, wobei, wenn irgendeiner von R¹, R² und R³ eine substituierte Alkylgruppe, eine substituierte Alkenylgruppe, eine substituierte Alkinylgruppe, eine substituierte aromatische Ringgruppe, eine substituierte Alkylcarbonylgruppe, eine substituierte Arylcarbonylgruppe, eine substituierte Alkoxycarbonylgruppe, eine substituierte Alkylaminocarbonylgruppe oder eine substituierte Arylaminocarbonylgruppe ist oder wenn X eine substituierte Alkylcarbonyloxygruppe ist, irgendeines der Kohlenstoffatome substituiert sein kann und der Substituent aus der Gruppe ausgewählt wird, die aus Halogenatomen, einer Azidgruppe, einer Nitrogruppe, Niederalkylgruppen, Niederhaloalkylgruppen, Niederalkoxygruppen, Niederhaloalkoxygruppen, Niederalkylaminogruppen, Niederalkylthiogruppen, einer Cyanogruppe, Niederalkoxycarbonylgruppen, einer Aminocarbonylgruppe, Niederalkylaminocarbonylgruppen, Niederalkenylgruppen, Niederalkinylgruppen, aromatischen Ringgruppen, aromatischen Ring-Oxy-Gruppen, aliphatischen heterocyclischen Gruppen, geschützten Hydroxylgruppen, geschützten Aminogruppen, geschützten Thiolgruppen, einer Aldehydgruppe, geschützten Aldehydgruppen, geschützten Carboxylgruppen und Kombinationen davon besteht, wobei der Begriff "Nieder" bedeutet, dass die Gruppe, an welche der Begriff angefügt ist, 1 bis 6 Kohlenstoffatome in der Form einer linearen Struktur, einer verzweigten Struktur oder einer cyclischen Struktur im Fall von 3 oder mehr Kohlenstoffatomen aufweist.

2. Verfahren zum Herstellen des Produktes der Hydroxylgruppensubstitution nach Anspruch 1, wobei ein optisch aktives Produkt einer Hydroxylgruppensubstitution der allgemeinen Formel [4] als das Produkt der Hydroxylgruppensubstitution durch Reagieren eines optisch aktiven Alkohols der allgemeinen Formel [3] mit Sulfurylfluorid (SO₂F₂) in der Gegenwart einer organischen Base und eines Nucleophils (Y⁻) hergestellt wird, 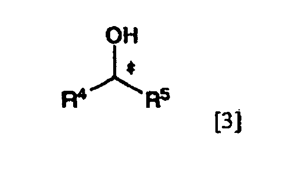 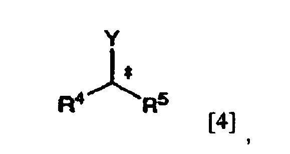 wobei R⁴ und R⁵ jeweils unabhängig eine Alkylgruppe, eine substituierte Alkylgruppe, eine Formylgruppe, eine Alkylcarbonylgruppe, eine substituierte Alkylcarbonylgruppe, eine Arylcarbonylgruppe, eine substituierte Arylcarbonylgruppe, eine Alkoxycarbonylgruppe, eine substituierte Alkoxycarbonylgruppe, eine Aminocarbonylgruppe, eine Alkylaminocarbonylgruppe, eine substituierte Alkylaminocarbonylgruppe, eine Arylaminocarbonylgruppe, eine substituierte Arylaminocarbonylgruppe oder eine Cyanogruppe darstellen; R⁴ und R⁵ in der Art voneinander verschieden sind; wenn R⁴ und R⁵ beliebige Substituentengruppen mit Ausnahme einer Formylgruppe, Aminocarbonylgruppe und Cyanogruppe sind, die Substituentengruppen eine Ringstruktur durch eine kovalente Bindung zwischen Kohlenstoffatomen davon durch oder ohne ein Heteroatom bilden können; Y ein Halogenatom, das aus der Gruppe ausgewählt wird, die aus Chlor, Brom und lod besteht, eine Azidgruppe, eine Formyloxygruppe, eine Alkylcarbonyloxygruppe, eine substituierte Alkylcarbonyloxygruppe, eine Arylcarbonyloxygruppe oder eine substituierte Arylcarbonyloxygruppe darstellt; * ein asymmetrisches Kohlenstoffatom darstellt; und die Konfiguration des asymmetrischen Kohlenstoffatoms bei der Reaktion umgekehrt wird, wobei, wenn irgendeiner von R⁴ und R⁵ eine substituierte Alkylgruppe, eine substituierte Alkylcarbonylgruppe, eine substituierte Arylcarbonylgruppe, eine substiuierte Alkoxycarbonylgruppe, eine substituierte Alkylaminocarbonlygruppe oder eine substituierte Arylaminocarbonylgruppe ist oder wenn Y eine substituierte Alkylcarbonyloxygruppe oder eine substituierte Arylcarbonyloxygruppe ist, irgendeines der Kohlenstoffatome substituiert sein kann und der Substituent aus der Gruppe ausgewählt wird, die aus Halogenatomen, einer Azidgruppe, einer Nitrogruppe, Niederalkylgruppen, Niederhaloalkygruppen, Niederalkoxygruppen, Niederhaloalkoxygruppen, Niederalkylaminogruppen, Niederalkylthiogruppen, einer Cyanogruppe, Niederalkoxycarbonylgruppen, einer Aminocarbonylgruppe, Niederalkylaminocarbonylgruppen, Niederalkenylgruppen, Niederalkinylgruppen, aromatischen Ringgruppen, aromatischen Ring-Oxy-Gruppen, aliphatischen heterocyclischen Gruppen, geschützten Hydroxylgruppen, geschützten Aminogruppen, geschützten Thiolgruppen, einer Aldehydgruppe, geschützten Aldehydgruppen, geschützten Carboxylgruppen und Kombinationen davon besteht, wobei der Begriff "Nieder" bedeutet, dass die Gruppe, an welche der Begriff angefügt ist, 1 bis 6 Kohlenstoffatome in der Form einer linearen Struktur, einer verzweigten Struktur oder einer cyclischen Struktur im Fall von 3 oder mehr Kohlenstoffatomen aufweist.

3. Verfahren zum Herstellen des Produktes der Hydroxylgruppensubstitution nach Anspruch 2, wobei ein optisch aktiver Ester mit Substitution der α-Hydroxylgruppe der allgemeinen Formel [6] als das optisch aktive Produkt der Hydroxylgruppensubstitution durch Reagieren eines optisch aktiven α-Hydroxyesters der allgemeinen Formel [5] mit Sulfurylfluorid (SO₂F₂) in der Gegenwart einer organischen Base und eines Nucleophils (Z⁻) hergestellt wird, 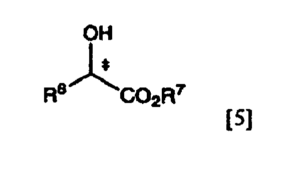 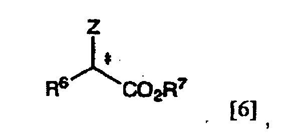 wobei R⁶ und R⁷ jeweils unabhängig eine Alkylgruppe oder eine substituierte Alkylgruppe darstellen und eine Ringstruktur durch eine kovalente Bindung zwischen Kohlenstoffatomen davon durch oder ohne ein Heteroatom bilden können; Z ein Halogenatom, das aus der Gruppe ausgewählt wird, die aus Chlor, Brom und lod besteht, oder eine Azidgruppe darstellt; * ein asymmetrisches Kohlenstoffatom darstellt; und die Konfiguration des asymmetrischen Kohlenstoffatoms bei der Reaktion umgekehrt wird, wobei, wenn irgendeiner von R⁶ und R⁷ eine substituierte Alkylgruppe ist, der Substituent aus der Gruppe ausgewählt wird, die aus Halogenatomen, einer Azidgruppe, einer Nitrogruppe, Niederalkylgruppen, Niederhaloalkylgruppen, Niederalkoxygruppen, Niederhaloalkoxygruppen, Niederalkylaminogruppen, Niederalkylthiogruppen, einer Cyanogruppe, Niederalkoxycarbonylgruppen, einer Aminocarbonylgruppe, Niederalkylaminogruppen, Niederalkenylgruppen, Niederalkinylgruppen, aromatischen Ringgruppen, aromatischen Ring-Oxy-Gruppen, aliphatischen heterocyclischen Gruppen, geschützten Hydroxylgruppen, geschützten Aminogruppen, geschützten Thiolgruppen, einer Aldehydgruppe, geschützten Aldehydgruppen, geschützten Carboxylgruppen und Kombinationen davon besteht, wobei der Begriff "nieder" bedeutet, dass die Gruppe, an welche der Begriff angefügt ist, 1 bis 6 Kohlenstoffatome in der Form einer linearen Struktur, einer verzweigten Struktur oder einer cyclischen Struktur im Fall von 3 oder mehr Kohlenstoffatomen aufweist.

4. Verfahren zum Herstellen des Produktes der Hydroxylgruppensubstitution nach Anspruch 2, wobei ein optisch aktives Prolin mit Substitution der 4-Hydroxylgruppe der allgemeinen Formel [8] als das optisch aktive Produkt der Hydroxylgruppensubstitution durch Reagieren eines optisch aktiven 4-Hydroxyprolins der allgemeinen Formel [7] mit Sulfurylfluorid (SO₂F₂) in der Gegenwart einer organischen Base und eines Nucleophils (Z⁻) hergestellt wird, 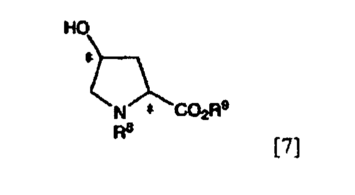 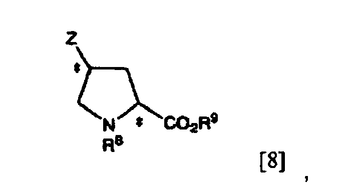 wobei R⁸ eine Schutzgruppe für ein sekundäres Amin darstellt; R⁹ eine Schutzgruppe für Carboxyl darstellt; Z ein Halogenatom, das aus der Gruppe ausgewählt wird, die aus Chlor, Brom und lod besteht, oder eine Azidgruppe darstellt; und * jeweils ein asymmetrische Kohlenstoffatom darstellt; die Konfiguration des asymmetrischen Kohlenstoffatoms an der 2-Position durchgehend bei der Reaktion aufrechterhalten wird; und die Konfiguration des asymmetrischen Kohlenstoffatoms an der 4-Position bei der Reaktion umgekehrt wird.

5. Verfahren zum Herstellen des Produkts der Hydroxylgruppensubstitution nach einem der Ansprüche 1 bis 3, wobei der Substituent eine Aminosäuregruppe oder eine Peptidgruppe als geschützte Aminogruppe ist.

## Revendications

1. Procédé de fabrication d'un produit de substitution par groupe hydroxyle de la formule générale [2], comprenant le fait de : faire réagir un alcool de la formule générale [1] avec du fluorure de sulfuryle (SO₂F₂) en présence d'une base organique et d'un nucléophile (X⁻) 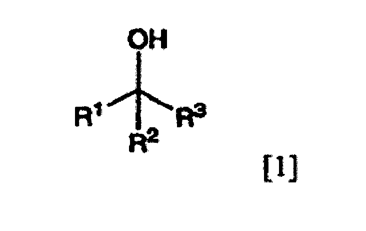 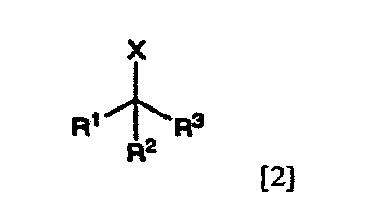 formules dans lesquelles R¹, R² et R³ représentent chacun indépendamment un atome d'hydrogène, un groupe alkyle, un groupe alkyle substitué, un groupe alcényle, un groupe alcényle substitué, un groupe alcynyle, un groupe alcynyle substitué, un groupe à noyau aromatique, un groupe à noyau aromatique substitué, un groupe formyle, un groupe alkylcarbonyle, un groupe alkylcarbonyle substitué, un groupe arylcarbonyle, un groupe arylcarbonyle substitué, un groupe alkoxycarbonyle, un groupe alkoxycarbonyle substitué, un groupe aminocarbonyle, un groupe alkylaminocarbonyle, un groupe alkylaminocarbonyle substitué, un groupe arylaminocarbonyle, un groupe arylaminocarbonyle substitué ou un groupe cyano ; lorsque deux de R¹, R² et R³ représentent des groupes substituants quelconques autres que l'atome d'hydrogène, le groupe formyle, le groupe aminocarbonyle et le groupe cyano, ces deux groupes substituants peuvent former une structure à noyau par une liaison covalente entre des atomes de carbone de ceux-ci par l'intermédiaire ou non d'un hétéroatome ; et X représente un atome d'halogène choisi dans le groupe consistant en des atomes de chlore, brome et iode, un groupe azide, un groupe nitrooxy, un groupe cyano, un groupe thiocyanate, un groupe formyloxy, un groupe alkylcarbonyloxy, un groupe alkylcarbonyloxy substitué, un groupe arylcarbonyloxy ou un groupe arylcarbonyloxy substitué, où, si l'un de R¹, R² et R³ représente un groupe alkyle substitué, un groupe alcényle substitué, un groupe alcynyle substitué, un groupe à noyau aromatique substitué, un groupe alkylcarbonyle substitué, un groupe arylcarbonyle substitué, un groupe alkoxycarbonyle substitué, un groupe alkylaminocarbonyle substitué ou un groupe arylaminocarbonyle substitué ou si X représente un groupe alkylcarbonyloxy substitué, l'un des atomes de carbone peut être substitué, et le substituant est choisi dans le groupe consistant en des atomes d'halogène ; un groupe azide ; un groupe nitro ; des groupes alkyle inférieurs ; des groupes halogénalkyle inférieurs ; des groupes alkoxy inférieurs ; des groupes halogénalkoxy inférieurs ; des groupes alkylamino inférieurs ; des groupes alkylthio inférieurs ; un groupe cyano ; des groupes alkoxycarbonyle inférieurs ; un groupe aminocarbonyle ; des groupes alkylaminocarbonyle inférieurs ; des groupes alcényle inférieurs ; des groupes alcynyle inférieurs ; des groupes à noyau aromatique ; des groupes oxy à noyau aromatique ; des groupes hétérocycliques aliphatiques ; des groupes hydroxyle protégés ; des groupes amino protégés ; des groupes thiol protégés ; un groupe aldéhyde ; des groupes aldéhyde protégés ; des groupes carboxyle protégés et des combinaisons de ceux-ci, où le terme « inférieur » signifie que le groupe auquel le terme est lié contient 1 à 6 atomes de carbone sous la forme d'une structure linéaire, d'une structure ramifiée ou d'une structure à noyau dans le cas de 3 ou plus de 3 atomes de carbone.

2. Procédé de fabrication d'un produit de substitution par groupe hydroxyle selon la revendication 1, dans lequel un produit de substitution par groupe hydroxyle optiquement actif de la formule générale [4] est fabriqué comme étant le produit de substitution par groupe hydroxyle en faisant réagir un alcool optiquement actif de la formule générale [3] avec du fluorure de sulfuryle (SO₂F₂) en présence d'une base organique et d'un nucléophile (Y⁻), 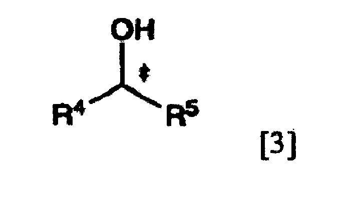 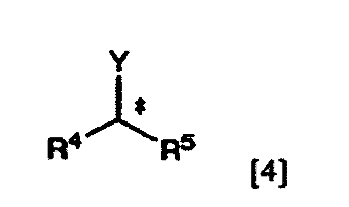 formules dans lesquelles R⁴ et R⁵ représentent chacun indépendamment un groupe alkyle, un groupe alkyle substitué, un groupe formyle, un groupe alkylcarbonyle, un groupe alkylcarbonyle substitué, un groupe arylcarbonyle, un groupe arylcarbonyle substitué, un groupe alkoxycarbonyle, un groupe alkoxycarbonyle substitué, un groupe aminocarbonyle, un groupe alkylaminocarbonyle, un groupe alkylaminocarbonyle substitué, un groupe arylaminocarbonyle, un groupe arylaminocarbonyle substitué, ou un groupe cyano ; R⁴ et R⁵ sont de nature différente l'un de d'autre ; lorsque R⁴ et R⁵ représentent des groupes substituants quelconques autres que le groupe formyle, le groupe aminocarbonyle et le groupe cyano, les groupes substituants peuvent former une structure à noyau par une liaison covalente entre les atomes de carbone de ceux-ci par l'intermédiaire ou non d'un hétéroatome ; Y représente un atome d'halogène choisi dans le groupe consistant en des atomes de chlore, brome et iode, un groupe azide, un groupe formyloxy, un groupe alkylcarbonyloxy, un groupe alkyl-carbonyloxy substitué, un groupe arylcarbonyloxy ou un groupe arylcarbonyloxy substitué ; * représente un atome de carbone asymétrique ; et la configuration de l'atome de carbone asymétrique est inversée dans la réaction, où, si l'un de R⁴ et R⁵ représente un groupe alkyle substitué, un groupe alkylcarbonyle substitué, un groupe arylcarbonyle substitué, un groupe alkoxycarbonyle substitué, un groupe alkylaminocarbonyle substitué ou un groupe arylamino-carbonyle substitué ou si Y représente un groupe alkylcarbonyloxy substitué ou un groupe arylcarbonyloxy substitué, l'un des atomes de carbone peut être substitué, et le substituant est choisi dans le groupe consistant en des atomes d'halogène ; un groupe azide ; un groupe nitro ; des groupes alkyle inférieurs ; des groupes halogénalkyle inférieurs ; des groupes alkoxy inférieurs ; des groupes halogénalkoxy inférieurs ; des groupes alkylamino inférieurs ; des groupes alkylthio inférieurs ; un groupe cyano ; des groupes alkoxycarbonyle inférieurs ; un groupe aminocarbonyle ; des groupes alkylaminocarbonyle inférieurs ; des groupes alcényle inférieurs ; des groupes alcynyle inférieurs ; des groupes à noyau aromatique ; des groupes oxy à noyau aromatique ; des groupes hétérocycliques aliphatiques ; des groupes hydroxyle protégés ; des groupes amino protégés ; des groupes thiol protégés ; un groupe aldéhyde ; des groupes aldéhyde protégés ; des groupes carboxyle protégés et des combinaisons de ceux-ci, où le terme « inférieur » signifie que le groupe auquel le terme est lié contient 1 à 6 atomes de carbone sous la forme d'une structure linéaire, d'une structure ramifiée ou d'une structure à noyau dans le cas de 3 ou plus de 3 atomes de carbone.

3. Procédé de fabrication d'un produit de substitution par groupe hydroxyle selon la revendication 2, dans lequel un ester de substitution par groupe α-hydroxyle optiquement actif de la formule générale [6] est fabriqué comme étant le produit de substitution par groupe hydroxyle optiquement actif en faisant réagir un α-hydroxyester optiquement actif de la formule générale [5] avec du fluorure de sulfuryle (SO₂F₂) en présence d'une base organique et d'un nucléophile (Z⁻), 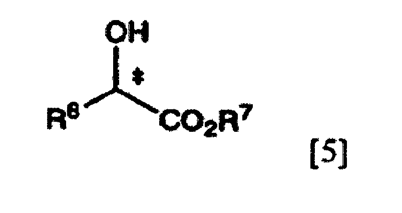 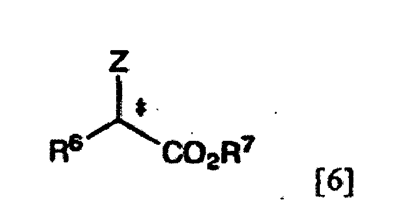 formules dans lesquelles R⁶ et R⁷ représentent chacun indépendamment un groupe alkyle ou un groupe alkyle substitué et peuvent former une structure à noyau par une liaison covalente entre des atomes de carbone de ceux-ci par l'intermédiaire ou non d'un hétéroatome ; Z représente un atome d'halogène choisi dans le groupe consistant en des atomes de chlore, brome et iode ou un groupe azide ; * représente un atome de carbone asymétrique ; et la configuration de l'atome de carbone asymétrique est inversée dans la réaction, où, si l'un de R⁶ et R⁷ est un groupe alkyle substitué, le substituant est choisi dans le groupe consistant en des atomes d'halogène ; un groupe azide ; un groupe nitro ; des groupes alkyle inférieurs ; des groupes halogénalkyle inférieurs ; des groupes alkoxy inférieurs ; des groupes halogénalkoxy inférieurs ; des groupes alkylamino inférieurs ; des groupes alkylthio inférieurs ; un groupe cyano ; des groupes alkoxycarbonyle inférieurs ; un groupe aminocarbonyle ; des groupes alkylaminocarbonyle inférieurs ; des groupes alcényle inférieurs ; des groupes alcynyle inférieurs ; des groupes à noyau aromatique ; des groupes oxy à noyau aromatique ; des groupes hétérocycliques aliphatiques ; des groupes hydroxyle protégés ; des groupes amino protégés ; des groupes thiol protégés ; un groupe aldéhyde ; des groupes aldéhyde protégés ; des groupes carboxyle protégés et des combinaisons de ceux-ci, où le terme « inférieur » signifie que le groupe auquel le terme est lié contient 1 à 6 atomes de carbone sous la forme d'une structure linéaire, d'une structure ramifiée ou d'une structure à noyau dans le cas de 3 ou plus de 3 atomes de carbone.

4. Procédé de fabrication d'un produit de substitution par groupe hydroxyle selon la revendication 2, dans lequel une proline de substitution par groupe 4-hydroxyle optiquement actif de la formule générale [8] est fabriquée comme étant le produit de substitution par groupe hydroxyle optiquement actif en faisant réagir une 4-hydroxyproline optiquement active de la formule générale [7] avec du fluorure de sulfuryle (SO₂F₂) en présence d'une base organique et d'un nucléophile (Z⁻), 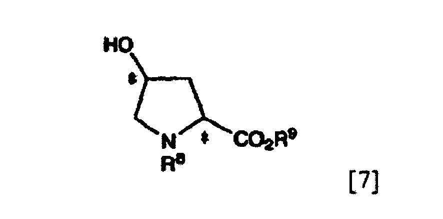 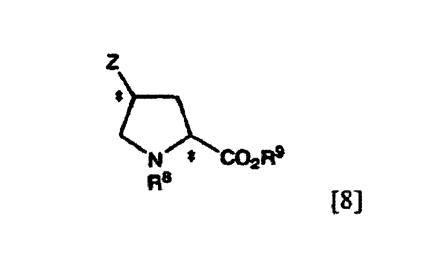 formules dans lesquelles R⁸ représente un groupe protecteur de la fonction amino secondaire ; R⁹ représente un groupe protecteur de la fonction carboxyle ; Z représente un atome d'halogène choisi dans le groupe consistant en des atomes de chlore, brome et iode ou un groupe azide ; et * représentent chacun un atome de carbone asymétrique ; la configuration de l'atome de carbone asymétrique en position 2 est maintenue tout au long de la réaction ; et la configuration de l'atome de carbone asymétrique en position 4 est inversée dans la réaction.

5. Procédé de fabrication du produit de substitution par groupe hydroxyle selon l'une des revendications 1 à 3, dans lequel le substituant est un groupe amino-acide ou un groupe peptide comme un groupe amino protégé.
